(19) Europäisches Patentamt
European Patent Office
Office européen des brevets

(11) EP 1 177 163 B1

(12) **EUROPÄISCHE PATENTSCHRIFT**

(45) Veröffentlichungstag und Bekanntmachung des Hinweises auf die Patenterteilung:
**04.02.2004 Patentblatt 2004/06**

(21) Anmeldenummer: **00925222.2**

(22) Anmeldetag: **18.04.2000**

(51) Int Cl.[7]: **C07C 45/50**, C07C 47/02

(86) Internationale Anmeldenummer:
**PCT/EP2000/003500**

(87) Internationale Veröffentlichungsnummer:
**WO 2000/066526 (09.11.2000 Gazette 2000/45)**

# (54) VERFAHREN ZUR HERSTELLUNG VON ALDEHYDEN

METHOD FOR PRODUCING ALDEHYDES

PROCEDE DE PRODUCTION D'ALDEHYDES

(84) Benannte Vertragsstaaten:
**AT BE CH CY DE DK ES FI FR GB GR IE IT LI LU MC NL PT SE**

(30) Priorität: **29.04.1999 DE 19919495**

(43) Veröffentlichungstag der Anmeldung:
**06.02.2002 Patentblatt 2002/06**

(73) Patentinhaber: **Celanese Chemicals Europe GmbH**
**60439 Frankfurt am Main (DE)**

(72) Erfinder:
- **BAHRMANN, Helmut**
  **D-46499 Hamminkeln (DE)**
- **BOHEN, Hans**
  **D-47441 Moers (DE)**

(56) Entgegenhaltungen:
**EP-A- 0 435 084**
**EP-A- 0 776 880**
**EP-A- 0 924 182**

EP 1 177 163 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft ein Verfahren zur Herstellung von Aldehyden durch Umsetzung von Olefinen oder olefinisch ungesättigten Verbindungen mit Wasserstoff und Kohlenmonoxid (Hydroformylierung) in Gegenwart von Rhodium oder Rhodiumverbindungen und einer nichtwäßrigen ionischen Flüssigkeit der allgemeinen Formel $(Q^+)_aA^{a-}$. In dieser Formel steht $Q^+$ für ein einfach geladenes, gegebenenfalls durch organische Reste substituiertes Ammonium-Kation oder das Äquivalent eines mehrfach geladenen, gegebenenfalls durch organische Reste substituierten Ammonium-Kations und $A^{a-}$ für das Anion eines sulfonierten oder carboxylierten Triesters der phosphorigen Säure. a ist eine ganze Zahl, mindestens gleich 1, und beschreibt die Ladung des Anions bzw. die Anzahl der Kationen mit der Ladung +1 in den der allgemeinen Formel entsprechenden Verbindungen.

Aldehyde besitzen als wertvolle Zwischenprodukte in der technischen Chemie eine große wirtschaftliche Bedeutung. Aus ihnen lassen sich z.B. Alkohole, Carbonsäuren und Amine herstellen, die wiederum als Ausgangsstoffe für die Herstellung wichtiger Endprodukte verwendet werden.

Die Hydroformylierung gehört zu den industriell in großem Umfang ausgeübten Verfahren. Die Reaktion wird durch Hydridometallcarbonyle, vorzugsweise solcher der Metalle der Gruppe VIII des Periodensystems der Elemente, katalysiert. Während zunächst ausschließlich Kobalt als Katalysatormetall technische Anwendung fand, gewinnen nunmehr Prozesse, die Rhodium als Katalysatormetall einsetzen, zunehmende Bedeutung.

Die Herstellung von Aldehyden durch Hydroformylierung von Olefinen kann einphasig in einer organischen Phase erfolgen. Dabei liegt der Katalysator, z.B. ein Rhodium/Triphenylphosphinkomplex, gelöst in dem aus Ausgangsstoffen und Reaktionsprodukt gebildeten Reaktionsgemisch vor. Zusätzlich kann ein organisches Lösungsmittel, z.B. Toluol, Xylol oder Tetrahydrofuran, zugegen sein.

EP-A1-0 435 084 betrifft die Hydroformylierung in homogener Phase in Gegenwart von Rhodium und in organischen Medien lösliches Ammoniumsalz eines sulfonierten Phosphorigsäuretriesters. Als Ammoniumionen werden Kationen der allgemeinen Formel $[NH_xR_y^1]^+$ eingesetzt.

Probleme wirft bei diesem Verfahren die Abtrennung der Reaktionsprodukte und die Wiedergewinnung der im Reaktionsprodukt homogen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und der daraus resultierenden Bildung von Nebenprodukten zu Lasten der Aldehydausbeute aber nur bei der Hydroformylierung niedriger Olefine, d.h. Olefinen mit bis zu etwa 5 Kohlenstoffatomen im Molekül, beschritten werden. Außerdem kann die thermische Belastung des Destillationsgutes zu erheblichen Verlusten an Katalysator durch Zersetzung der katalytisch wirksamen Komplexverbindungen führen.

Diese Mängel lassen sich vermeiden, wenn die Hydroformylierungsreaktion in einem Zweiphasensystem durchgeführt wird. Ein derartiges Verfahren ist z.B. in der DE-PS 26 27 354 beschrieben. Dieser Prozeß ist charakterisiert durch das Vorliegen einer organischen Phase, die die Ausgangsolefine und das Reaktionsprodukt enthält und einer wäßrigen Phase, in der der Katalysator gelöst ist. Als Katalysatoren werden wasserlösliche Rhodiumkomplexverbindungen eingesetzt, die wasserlösliche Phosphine als Liganden enthalten. Zu den Phosphinen zählen insbesondere Triarylphosphine, Trialkylphosphine und arylierte bzw. alkylierte Diphosphine, deren organische Reste durch Sulfonsäuregruppen oder Carboxylgruppen substituiert sind. Ihre Herstellung ist z.B. aus DE-PS 26 27 354 bekannt.

Der in Gegenwart einer wäßrigen katalysatorhaltigen Phase zweiphasig durchgeführte Hydroformylierungsprozeß bewährt sich besonders bei der Hydroformylierung niedriger Olefine, insbesondere bei Ethylen und Propylen. Setzt man hingegen höhere Olefine wie Hexen, Octen oder Decen ein, so geht der Umsatz deutlich zurück. Die Minderung des Umsatzes ist wohl auf die Abnahme der Löslichkeit höherer Olefine in Wasser zurückzuführen, da man annimmt, daß die Reaktion zwischen den Reaktanten in der wäßrigen Phase abläuft. Für diese Hypothese spricht, daß der Olefinumsatz deutlich erhöht wird, wenn man der wäßrigen Katalysatorlösung ein Phasentransferreagenz (Lösungsvermittler) zusetzt. Bewährt haben sich nach EP-B-0 562 451 insbesondere kationische Lösungsvermittler der allgemeinen Formel $[A-N(R^1R^2R^3)]^+ E^-$, in der A für einen geradkettigen oder verzweigten Alkylrest mit 6 bis 25 Kohlenstoffatomen steht, $R^1$, $R^2$, $R^3$ gleich oder verschieden sind und geradkettige oder verzweigte Alkylreste mit 1 bis 4 Kohlenstoffatomen bedeuten und $E^-$ für ein Anion, insbesondere Sulfat, Tetrafluoroborat, Acetat, Methosulfat, Benzolsulfonat, Alkylbenzolsulfonat, Toluolsulfonat, Lactat oder Citrat steht.

Neben der ausreichenden Löslichkeit des Olefins in der wäßrigen Phase ist für die zweiphasige Durchführung des Hydroformylierungsprozesses in Gegenwart einer wäßrigen katalysatorhaltigen Phase eine ausreichenden Stabilität des umzusetzenden Olefins gegenüber Wasser notwendig. Wasserempfindliche Olefine, wie z.B. Acrylsäureester oder ungesättigte Acetale, lassen sich daher nach diesem Verfahren nicht erfolgreich einsetzen.

Um den geschilderten Nachteil zu überwinden, ohne auf den Vorteil des zweiphasig durchgeführten Hydroformylierungsprozesses verzichten zu müssen, wird die Verwendung von unpolaren Perfluorkohlenwasserstoffen, z. B. von Perfluormethylcyclohexan als nichtwäßrige, nicht mit dem organischen Reaktionsprodukt mischbaren Phase für die katalytische Hydroformylierung von Olefinen vorgeschlagen. Jedoch sind zum Lösen der Rhodiumkomplexe in den Perfluorkohlenwasserstoffen spezielle fluorierte Liganden, wie zum Beispiel Tris-(1H,1H,2H-perfluoroctyl)phosphan notwendig (Science 1994,266,72).

Eine andere Arbeitsweise, katalytische Reaktionen in einem nichtwäßrigen Zweiphasensystem durchzuführen, wird in CHEMTECH, September 1995, Seiten 26 bis 30 beschrieben. Hiernach werden nichtwäßrige ionische Flüssigkeiten, die schon bei Raumtemperatur flüssig sind, z.B. eine Mischung aus 1,3-Dialkylimidazolium Chlorid, bevorzugt 1-n-Butyl-3-methylimidazolium Chlorid (abgekürzt $[BMI]^+[Cl]^-$), und Aluminiumchlorid und/oder Ethylaluminiumdichlorid, als Lösungsmittel für den Katalysator verwendet. Als Beispiele für mit derartigen Katalysatorlösungen durchgeführte Reaktionen sind die Olefindimerisierung in Gegenwart von Nickelkomplexverbindungen, z.B. die Propendimerisierung zu isomeren Hexenen oder die Butendimerisierung zu Iso-Octenen zu nennen. Bei diesen Umsetzungen fällt das Reaktionsprodukt als obere Phase an, während die katalysatorhaltige nichtwäßrige ionische Flüssigkeit die untere Phase bildet und durch einfache Phasentrennung isoliert werden kann. Die Lösung des Katalysators in der nichtwäßrigen ionischen Flüssigkeit kann erneut in den Prozeß eingeführt werden.

Aus Am. Chem. Soc., Div. Pet. Chem. 1992, 37, Seiten 780 bis 785 ist bekannt, daß eine nichtwäßrige ionische Flüssigkeit, bestehend aus $[BMI]^+[Cl]^-$ und Aluminiumchlorid als Lösungsmittel dient, in dem nach Zusatz von Ethylaluminiumdichlorid und $NiCl_2(PR_3)_2$, mit R gleich Isopropyl, die Dimerisierung von Propen erfolgt.

Die Verwendung von niedrig schmelzenden Phosphoniumsalzen, z.B. von Tetrabutylphosphonium-Bromid als Lösungsmittel in Hydroformylierungsreaktionen wird im Journal of Molecular Catalysis, 47 (1988) Seiten 99 - 116 beschrieben. Danach führt die Hydroformylierung von Olefinen, z.B.Octen-1, mit Rutheniumcarbonylkomplexen in Gegenwart von stickstoff- oder phosphorhaltigen Liganden, z.B. von 2,2'-Dipyridin oder 1,2-Bis(diphenylphosphino)ethan, bei Temperaturen von 120 bis 180 °C zu einem Gemisch aus n-Nonanol und n-Nonanal. Bei diesem Verfahren erhält man ein Reaktionsgemisch mit einem n-Nonanol-Anteil von bis zu 69 Gew.-%, bezogen auf das Reaktionsgemisch. Die Isolierung des gewünschten n-Nonanals erfordert daher einen erheblichen Destillationsaufwand.

Die europäische Patentanmeldung EP-A-0 776 880 offenbart die Hydroformylierung von Olefinen in Gegenwart von quatemären Ammonium- und/oder Phosphoniumsalzen als Lösungsmittel für den Katalysator. Bevorzugt werden Salze, die $[BMI]^+$ als Kation enthalten.

Auch Salze von quaternären Diaminen, bei denen das Kation die allgemeine Formel

$$R^1R^2N^{\oplus}{=}CR^3\text{-}R^5\text{-}R^3C{=}N^{\oplus}R^1R^2$$

hat, wobei $R^1$, $R^2$, $R^3$ gleich oder verschieden sind und Wasserstoff oder einen Kohlenwasserstoffrest mit 1 bis 12 Kohlenstoffatomen bedeuten und $R^5$ ein Alkylenrest, z.B. Methylen ($-CH_2-$), Ethylen ($-CH_2-CH_2-$), Propylen ($-CH_2-CH_2-CH_2-$) oder ein Phenylenrest, ist, werden als Lösungsmittel für Hydroformylierungskatalysatoren verwendet. Geeignete Anionen sind beispielsweise Hexafluorophosphat, Hexafluoroantimonat, Tetrachloroaluminat oder Tetrafluoroborat. Diese quatemären Ammonium- und/oder Phosphoniumsalze sind bereits unterhalb von 90°C, vorzugsweise unterhalb von 85°C und besonders bevorzugt unterhalb von 50°C flüssig.

Der in den genannten Lösungsmitteln gelöste Hydroformylierungskatalysator enthält als aktives Metall Kobalt, Rhodium, Iridium, Ruthenium, Palladium oder Platin und als Ligand ein tertiäres Phosphin oder tertiäres sulfoniertes Phosphin, ein tertiäres Arsin, tertiäres Stilbin oder ein Phosphit. Das Molverhältnis von Ligand zu Metall beträgt 9,5.

Die katalytisch wirksamen Metalle werden als Verbindungen, Rhodium z.B. in Form von Rhodiumacetylacetonatdicarbonyl oder Rhodiumcarbonyl $Rh_6(CO)_{16}$, eingesetzt. Aus ihnen bildet sich unter den Reaktionsbedingungen der Hydroformylierungskatalysator. Die Hydroformylierungsreaktion wird besonders bevorzugt zwischen 30 und 90°C durchgeführt.

Auch nach Angew. Chem. 1995, 107 Nr.23/24 Seiten 2941 bis 2943 lassen sich Hydroformylierungsreaktionen unter Verwendung bei Raumtemperatur flüssiger 1,3 Dialkylimidazoliumsalze als katalysatorhaltiges, nicht mit dem organischen Reaktionsgemisch mischbares Lösungsmittel durchführen. Hierzu wird Rhodiumdicarbonylacetylacetonat als Katalysatorvorstufe zu einer Lösung von Triphenylphosphin in $[BMI]^{\oplus}[PF_6]^{\ominus}$ gegeben, das Molverhältnis Phosphor (III) zu Rhodium kann von 3 bis 10 variieren. Der Katalysator wird mit Synthesegas (Volumenverhältnis Wasserstoff zu Kohlenmonoxid gleich 1:1) präformiert. Anschließend setzt man n-Penten-1 mit Synthesegas gleicher Zusammensetzung bei einer Temperatur von 80°C um. Auch in diesem Falle läßt sich die organische Produktphase in einfacher Weise von der katalysatorhaltigen, nichtwäßrigen ionischen Flüssigkeit durch Dekantieren abtrennen.

Die bekannten Verfahren zur Hydroformylierung von Olefinen sind durch die Verwendung einer nichtwäßrigen ionischen Flüssigkeit, die als Lösungsmittel für den katalytisch wirksamen Metallkomplex dient, charakterisiert. Durch die Verwendung der nichtwäßrigen ionischen Flüssigkeit als Lösungsmittel werden zusätzliche, nicht als Liganden dienende Anionen, z.B. Hexafluoroantimonat oder Hexafluorophosphat, in den Hydroformylierungsprozeß eingebracht.

Weiterhin folgt aus dem Stand der Technik (vgl. Angew. Chem. 1995, 107 Nr.23/24 Seiten 2941 bis 2943 und EP-A-0 776 880), daß sich das Molverhältnis Phosphor zu Rhodium im Bereich von 3 bis 10 bewegt. Höhere Molverhältnisse werden offensichtlich als ungeeignet angesehen, obgleich eine Vergrößerung des Ligandanteils, bezogen

auf das Metall, die Stabilität der katalytisch wirksamen Komplexverbindung verbessern sollte. Möglicherweise ist die Löslichkeit der als Liganden wirkenden Verbindungen in den bisher gebräuchlichen ionischen Flüssigkeiten begrenzt, so daß sie sich bei Überschreiten einer maximalen Konzentration aus der Lösung abscheiden und aus der Katalysatorphase ausgetragen werden.

Nachteilig bei den bekannten Verfahren ist neben dem Austrag des Phosphinliganden der Austrag des katalytisch aktiven Metalls aus der nichtwäßrigen ionischen Flüssigkeit in die organische Phase. Nach dem Stand der Technik läßt sich dieser Nachteil umgehen, wenn an Stelle von neutralen Liganden, wie Triphenylphosphin, geladene Liganden, z.B. mono- oder trisulfoniertes Triphenylphosphin, verwendet werden, da zu erwarten ist, daß geladene Liganden die Löslichkeit der katalytisch aktiven Metallverbindungen in der nichtwäßrigen ionischen Flüssigkeit erhöhen. Zwar konnte durch Verwendung geladener Liganden der Austrag des katalytisch aktiven Metalls reduziert werden, gleichzeitig verringerte sich jedoch die Aldehydausbeute auf nur noch 16 bis 33 % (Angew. Chem. 1995, 107 Nr.23/24 Seiten 2941 bis 2943, EP-A-0 776 880).

Es bestand daher die Aufgabe ein Verfahren zur Hydroformylierung von Olefinen oder olefinisch ungesättigten Verbindungen in Gegenwart eines katalytisch wirkenden Metalls und einer nichtwäßrigen ionischen Flüssigkeit zu entwickeln, das die beschriebenen Nachteile vermeidet, insbesondere die Reaktionsprodukte - Aldehyde und Verbindungen, die Aldehydgruppen enthalten - in hohen Ausbeuten liefert, wobei Katalysatorverluste nicht oder allenfalls in untergeordnetem, vertretbarem Maße auftreten.

Die Erfindung besteht in einem zweiphasigen Verfahren zur Herstellung von Aldehyden durch Umsetzung von Monoolefinen, nichtkonjugierten Polyolefinen, Cycloolefinen oder Derivaten dieser Verbindungsklassen mit Kohlenmonoxid und Wasserstoff bei Temperaturen von 20 bis 150°C und Drücken von 0,1 bis 20 MPa, charakterisiert durch das Vorliegen einer organischen aldehydhaltigen Phase und einer darin nicht löslichen nichtwäßrigen ionischen Flüssigkeit der allgemeinen Formel $(Q^{\oplus})_a A^{a-}$ mit mindestens einer Rhodiumverbindung. Es ist dadurch gekennzeichnet, daß $Q^{\oplus}$ für von Monoaminen abgeleitete Ammoniumionen der allgemeinen Formel (5)

$$R^3R^4N^{\oplus} = CR^5R^6 \qquad (5)$$

steht, wobei $R^3$, $R^4$, $R^5$, $R^6$ gleich oder verschieden sind und Wasserstoff, insbesondere mit der Maßgabe bedeuten, daß mindestens ein $R^3$, $R^4$, $R^5$, $R^6$ nicht Wasserstoff ist, oder für einen linearen oder verzweigten aliphatischen Kohlenwasserstoffrest mit 1 bis 20 Kohlenstoffatomen, einen cycloaliphatischen oder aromatischen Kohlenwasserstoffrest mit 6 bis 20 Kohlenstoffatomen oder einen Alkoxyrest mit 1 bis 10 Kohlenstoffatomen stehen; oder für von Monoaminen abgeleitete Ammoniumionen der allgemeinen Formel (6)

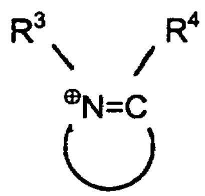

(6)

steht, die sich von gesättigten oder ungesättigten cyclischen Verbindungen sowie von aromatischen Verbindungen mit jeweils einem dreibindigen N-Atom im 4- bis 10-, vorzugsweise 5- bis 6-gliedrigen Ring ableiten, wobei $R^3$, $R^4$ gleich oder verschieden sind und Wasserstoff, einen linearen oder verzweigten aliphatischen Kohlenwasserstoffrest mit 1 bis 20 Kohlenstoffatomen, einen cycloaliphatischen oder aromatischen Kohlenwasserstoffrest mit 6 bis 20 Kohlenstoffatomen oder einen Alkoxyrest mit 1 bis 10 Kohlenstoffatomen bedeuten; oder für von Diaminen abgeleitete Ammoniumionen der allgemeinen Formel (7) oder (8)

$$R^3R^4R^5N^{\oplus}\text{-G-}N^{\oplus}R^6R^7R^8 \qquad (7)$$

$$R^3R^4N^{\oplus} = CR^5\text{-G-}R^5C = N^{\oplus}R^3R^4 \qquad (8)$$

steht, wobei $R^3$, $R^4$, $R^5$, $R^6$, $R^7$ und $R^8$ gleich oder verschieden sind und Wasserstoff, einen linearen oder verzweigten aliphatischen Kohlenwasserstoffrest mit 1 bis 20 Kohlenstoffatomen, einen cycloaliphatischen oder aromatischen Kohlenwasserstoffrest mit 6 bis 30 Kohlenstoffatomen, einen Alkylarylrest mit 7 bis 40 Kohlenstoffatomen oder einen Alkoxyrest mit 1 bis 10 Kohlenstoffatomen bedeuten, G für einen Alkylenrest $(-CHR^9-)_d$, wobei $R^9$ Wasserstoff oder ein Kohlenwasserstoffrest mit 1 bis 5 Kohlenstoffatomen und d eine ganze Zahl von 1 bis 8, vorzugsweise 2 bis 6 ist, für einen Arylenrest mit 6 bis 30 Kohlenstoffatomen oder einen Alkylenarylrest mit 7 bis 40 Kohlenstoffatomen, steht; oder für vom Imidazol und seinen Alkyl- und Phenylderivaten abgeleitete Kationen der allgemeinen Formel (10)

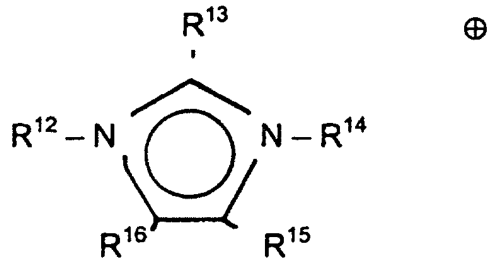

(10)

steht, in der $R^{12}$, $R^{13}$, $R^{14}$, $R^{15}$ und $R^{16}$ gleich oder verschieden sind und für Wasserstoff, einen $C_1$- bis $C_{30}$-Alkylrest, einen $C_6$- bis $C_{40}$-Arylrest, einen $C_7$-bis $C_{40}$-Alkylarylrest oder einen $SiR_3{}^{17}$-Rest, in dem $R^{17}$ einen $C_1$- bis $C_{30}$-Alkylrest oder einen $C_6$- bis $C_{40}$-Arylrest bedeutet, stehen, $A^{a-}$ für das Anion eines sulfonierten oder carboxylierten Triesters der phosphorigen Säure steht und a eine ganze Zahl und mindestens gleich 1 ist und dass die Aldehyd enthaltende Produktphase und die Rhodium und ionische Flüssigkeit enthaltende Katalysatorphase durch Phasentrennung voneinander getrennt werden und die Katalysatorphase partiell oder vollständig in den Hydroformylierungsprozeß zurückgeführt wird.

Überraschenderweise wurde gefunden, daß sich in den erfindungsgemäß eingesetzten nichtwäßrigen ionischen Flüssigkeiten gelöste Rhodiumverbindungen hervorragend für die Hydroformylierung von Olefinen oder olefinisch ungesättigten Verbindungen eignen. Ionische Flüssigkeit und Rhodiumverbindung bilden Katalysatorsysteme (eine Bezeichnung, die im folgenden beibehalten wird), die sich durch hohe Aktivität und Selektivität auszeichnen. Ihre bemerkenswerten Eigenschaften sind wahrscheinlich darauf zurückzuführen, daß Ester der phosphorigen Säure eine Doppelfunktion besitzen. Sie sind als ionische Flüssigkeiten nicht nur Lösungsmittel für die Metallverbindungen, sondern können als Liganden mit Rhodium als Zentralatom auch Komplexverbindungen (Koordinationsverbindungen) bilden. Daher wird für die ionischen Flüssigkeiten nachstehend auch der Begriff "Ligandflüssigkeit" verwendet. Das Vorhandensein von Liganden, gegebenenfalls auch in hohem Überschuß über die stöchiometrisch zur Komplexbildung erforderliche Menge, stabilisiert die katalytisch wirksame Metallverbindung, mit dem bereits beschriebenen Ergebnis verbesserten katalytischen Verhaltens. Darüber hinaus führt die Stabilisierung zu einer Verminderung der Edelmetallverluste, weil deutlich weniger Rhodium zusammen mit dem Reaktionsprodukt aus dem Reaktionsgemisch ausgetragen wird. Daher kann das Katalysatorsystem weitaus öfter in die Reaktionszonen zurückgeführt werden, als Rhodiumkatalysatoren in Abwesenheit einer Ligandflüssigkeit, ohne daß ein Absinken der Katalysatoraktivität und/oder -selektivität beobachtet wird. Stabilisierte Katalysatorsysteme ergeben höhere Ausbeuten an Aldehyden und weisen eine längere Lebensdauer auf als nicht stabilisierte Katalysatoren.

Von Bedeutung ist in diesem Zusammenhang auch, daß sich bei Verwendung nichtwäßriger ionischer Ligandflüssigkeiten im Hydroformylierungsprozeß sehr hohe P/Rh-Molverhältnisse einstellen lassen; sie können P:Rh = 1000:1 betragen und gegebenenfalls noch höher sein. Die Möglichkeit, die Phosphorverbindungen in sehr hohem Überschuß einzusetzen hat den Vorteil, daß Phosphorverluste durch Umwandlung und Abbau der Phosphorverbindungen, z.B. durch Übergang von P(III)-verbindungen in nicht koordinationsfähige P(V)-verbindungen im Verlauf der Hydroformylierungszyklen bei kontinuierlicher bzw. bei wiederholtem Katalysatoreinsatz im Falle diskontinuierlicher Reaktionsführung nicht ins Gewicht fallen. Denn einerseits liegt die Ligandflüssigkeit in so ausreichender Menge vor, daß, im Sinne des Massenwirkungsgesetzes, die Bildung der Phosphorliganden enthaltenden Koordinationsverbindungen sichergestellt ist. Andererseits erreichen die aus den Liganden resultierenden Folgeprodukte, bezogen auf die vorliegende Ligandflüssigkeit, nie eine so hohe Konzentration, daß sie schädigend in das Reaktionsgeschehen eingreifen können. Daher ist auch aus dieser Sicht eine rasche Erschöpfung des Katalysatorsystems nicht zu befürchten. Im übrigen können, sofern erforderlich, Fehlmengen an Metall und/oder Ligandflüssigkeit, durch Zuführen von frischem Katalysatorsystem oder seinen Komponenten, ausgeglichen werden.

Die gemäß der Erfindung eingesetzten Ammoniumsalze sulfonierter oder carboxylierter Phosphorigsäuretriester lassen sich formal von der phosphorigen Säure durch Veresterung mit den Ammoniumsalzen von Hydroxysulfonsäuren bzw. Hydroxycarbonsäuren der allgemeinen Formel

$$(Qac)_b\text{-}Y\text{-}(OH)_c \qquad (1)$$

ableiten, in der ac einen aciden Rest, nämlich den Sulfonsäurerest $-SO_3^-$ bzw. den Carbonsäurerest $-COO^-$ und Q die zuvor genannte Bedeutung besitzen.

Weiterhin steht in der allgemeinen Formel (1) Y für einen organischen Rest. Dementsprechend fallen unter diese Formel sulfonierte oder carboxylierte Hydroxyverbindungen, die sich von aliphatischen, cycloaliphatischen, aromatischen und heterocyclischen Grundstrukturen ableiten. Die aliphatischen Verbindungen können linear oder verzweigt und, wie die cycloaliphatischen Verbindungen, gesättigt oder ungesättigt sein. Zu den cycloaliphatischen und den aromatischen Verbindungen zählen sowohl einkernige als auch mehrkemige Strukturen. Ebenso gehören zu den Hydroxysäuren der erfindungsgemäß eingesetzten Phosphite aliphatisch-aromatische wie auch aromatisch-aliphatische Verbindungen. Als heterocyclische Verbindungen kommen gesättigte oder ungesättigte Ringsysteme mit Stickstoff, Sauerstoff oder Schwefel als Heteroatom in Betracht. Im Molekül können auch zwei oder mehr gleiche oder verschiedene Heteroatome enthalten sein. Überdies kann der Heterocyclus durch Alkylreste oder Arylreste substituiert oder mit weiteren Ringsystemen, aliphatischen, aromatischen oder heterocyclischen, kondensiert sein. Alle Verbindungen können noch weitere Substituenten tragen, von denen der Fachmann weiß, daß sie sich bei ihrer speziellen Anwendung als ionische Flüssigkeit inert verhalten.

Insbesondere steht Y in der obigen Formel (1) für lineare oder verzweigte, gesättigte aliphatische Reste mit insgesamt 1 bis 20 Kohlenstoffatomen, die durch Hydroxy- oder durch Alkoxyreste mit 1 bis 10 Kohlenstoffatomen substituiert sein können. Y bedeutet weiterhin vorzugsweise gesättigte oder ungesättigte ein- oder mehrkernige cycloaliphatische Reste mit 5 bis 14 Kohlenstoffatomen im Ring oder den Ringen und ein- oder mehrkernige aromatische Reste mit 6 bis 14 Kohlenstoffatomen im Ring oder den Ringen. Sowohl die cycloaliphatischen als auch die aromatischen Reste können neben Sulfonsäure- oder Carbonsäureresten noch weitere Substituenten enthalten, nämlich Alkylreste mit 1 bis 20 Kohlenstoffatomen, Aryl-, Alkylaryl- oder Aralkylreste mit 6 bis 30 Kohlenstoffatomen und Cycloalkylreste mit 5 bis 14 Kohlenstoffatomen, ferner Hydroxygruppen sowie Alkoxyreste mit 1 bis 10 Kohlenstoffatomen. Die aromatischen Reste leiten sich vorzugsweise vom Benzol, vom Biphenyl, vom Naphthalin und vom Binaphthyl ab. Als Arylalkylrest hat sich vor allem der leicht zugängliche, gegebenenfalls substituierte Benzylrest bewährt. Alkylarylreste gehen bevorzugt auf Toluol, Ethylbenzol und die isomeren Xylole zurück. Unter den Heterocyclen sind Reste Stickstoff enthaltender, gesättigter oder ungesättigter Fünf- oder Sechsringe von Bedeutung, insbesondere Pyridin. b und c schließlich sind jeweils ganze Zahlen und haben jeweils mindestens den Wert 1, c ist insbesondere 1 oder 2.

Zu den sulfonierten oder carboxylierten Estern der phosphorigen Säure gemäß der Erfindung gehören insbesondere Verbindungen der allgemeinen Formel (2)

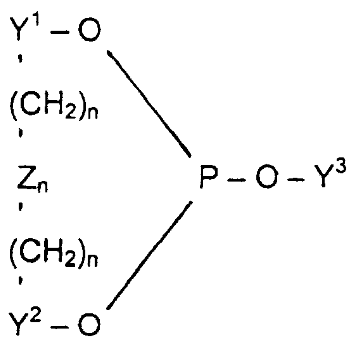

(2)

in der $Y^1$, $Y^2$ und $Y^3$ gleich oder verschieden sind, einen organischen Rest bedeuten und die für Y unter Formel (1) genannte Bedeutung haben. Z ist eine zweiwertige Brückengruppe und steht für $-CR^1R^2-$, wobei $R^1$ und $R^2$ unabhängig voneinander Wasserstoff oder Alkylreste mit 1 bis 12 Kohlenstoffatomen sind. Z steht weiterhin für -O-, -S-, -CO-, $-CH_2-CO-CH_2-$, n ist gleich oder verschieden und entspricht 0 oder 1 und im Falle Z gleich $-CR^1R^2-$ 1, 2 oder 3. Wenn n den Wert 0 hat, können die Reste $Y^1$, $Y^2$ und $Y^3$ selbständig sein. Zwei benachbarte Reste $Y^1$ und $Y^2$, $Y^2$ und $Y^3$ oder $Y^1$ und $Y^3$ können aber auch miteinander verbunden sein und z.B. einen bivalenten Rest bilden. Ist $Y^1$ und $Y^2$ jeweils ein sich vom Benzol ableitender Rest, so können diese beiden benachbarten Reste z.B. durch eine Einfachbindung zu einem bivalenten Biphenylrest verknüpft sein. Sofern benachbarte Reste $Y^1$, $Y^2$, $Y^3$ cycloaliphatische oder aromatische Strukturen bedeuten, können sie im Falle n = 0 auch linear ankondensiert (anneliert) sein. Es resultieren dann z.B. bivalente, gegebenenfalls substituierte cycloaliphatische, aromatische oder cycloaliphatisch-aromatische

Reste, z.B. zweibindige Dicyclodecylenoder Tricyclotetradecylenreste oder zweibindige Naphthylen- oder Anthracylenreste. Weiterhin enthalten die Ester der allgemeinen Formel (2) mindestens einen $ac^-$-Rest, also mindestens eine Sulfonsäuregruppe oder eine Carboxylgruppe.

In den Verbindungen der allgemeinen Formel (2) ist $Y^1$, $Y^2$, $Y^3$ vorzugsweise ein vom Benzol, vom Naphthalin, vom Biphenyl oder vom Binaphthyl abgeleiteter Rest, der jeweils durch einen oder mehrere Alkylreste mit 1 bis 20 Kohlenstoffatomen, durch einen oder mehrere Aryl-, Aralkyl-, Alkylarylreste mit 6 bis 30 Kohlenstoffatomen und/oder durch einen oder mehrere Cycloalkylreste mit 5 bis 14 Kohlenstoffatomen, durch Hydroxygruppen und/oder Alkoxyalkylreste mit 1 bis 10 Kohlenstoffatomen und/oder durch einen oder mehrere Säurereste ($-ac^-$) substituiert sein kann. Z steht insbesondere für den Rest $-CH_2-$, für -O-, -CO- und $-CH_2-O-CH_2-$.

Zu den der allgemeinen Formel (2) entsprechenden Verbindungen gehören Sulfonate oder Carboxylate von Trialkylphosphiten wie Trimethylphosphit, Triethylphosphit, Butyldiethylphosphit, Tri-n-propylphosphit, Tri-n-butylphosphit, Tri-2-ethylhexylphosphit, Tri-n-octylphosphit, Tri-n-dodecylphosphit, von Dialkylarylphosphiten wie Dimethylphenylphosphit, Diethylphenylphosphit, von Alkyldiarylphosphiten wie Methyldiphenylphosphit, Ethyldiphenylphosphit und von Triarylphosphiten wie Triphenylphosphit, Phenylbiphenylenphosphit und Trinaphthylphosphit.

Eine weitere Gruppe bedeutsamer sulfonierter oder carboxylierter Ester der phosphorigen Säure entsprechend der Erfindung sind Polyphosphite der allgemeinen Formel (3)

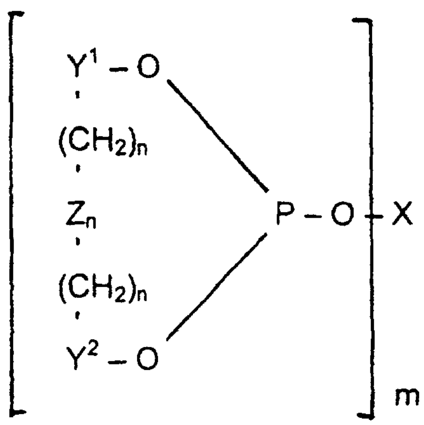

(3)

In dieser Formel sind $Y^1$ und $Y^2$ gleich oder verschieden und haben die unter Formel (1) für Y und unter Formel (2) für $Y^1$, $Y^2$ und $Y^3$ wiedergegebenen Bedeutungen. Die Definitionen von Z und n entsprechen den Angaben unter Formel (2). X steht für eine m-wertige Brückengruppe aus der Gruppe Alkylenreste, Alkylenoxyalkylenreste, Arylenreste oder Aryl-$Z_n$-arylreste. m ist eine ganze Zahl und hat einen Wert von 2 bis 6. Weiterhin enthält das Polyphosphit der allgemeinen Formel (3) mindestens einen $ac^-$-Rest, also mindestens eine Sulfonat-($-SO_3^-$) oder Carboxylat-($-COO^-$)-gruppe.

X wird bevorzugt durch Alkylenreste mit 2 bis 18, insbesondere 2 bis 12, Kohlenstoffatomen und durch Arylenreste mit 6 bis 18 Kohlenstoffatomen beschrieben. In der Bedeutung Aryl-$Z_n$-Aryl steht Z bevorzugt für $-CH_2-$, für -O-, -CO- und $-CH_2-CO-CH_2-$. Die durch X bezeichneten Reste können ebenfalls durch einen oder mehrere Alkyl- und/oder Alkoxyreste und/oder durch einen oder mehrere Säurereste ($-ac^-$) substituiert sein.

Weitere wichtige Vertreter der erfindungsgemäßen Ester der phosphorigen Säure werden durch die nachstehende allgemeine Formel (4) wiedergegeben.

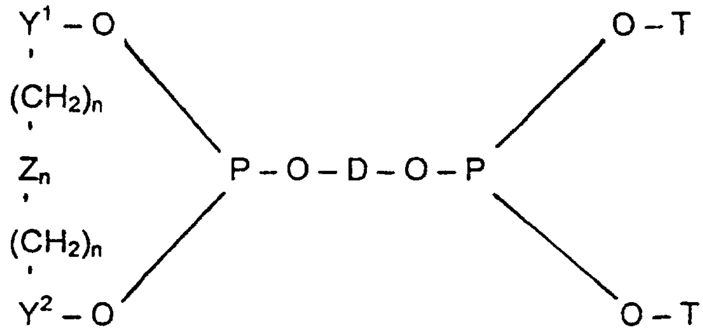

(4)

In dieser Formel sind $Y^1$ und $Y^2$ gleich oder verschieden und haben die unter Formel (1) für Y und unter Formel (3) für $Y^1$ und $Y^2$ wiedergegebene Bedeutung. Die Definitionen von Z und n entsprechen den Angaben unter Formel (2) und Formel (3). D steht für einen zweiwertigen Kohlenwasserstoffrest als Brückengruppe, nämlich einen Alkylenrest mit 1 bis 30 Kohlenstoffatomen, einen Arylen-, Alkylarylen-, Arylalkylenrest mit 6 bis 30 Kohlenstoffatomen und einen Aryl-$Z_n$-aryl-Rest. T ist ein einwertiger Kohlenwasserstoffrest mit 1 bis 30 Kohlenstoffatomen und kann ein Alkyl-, Aryl-, Aralkyl-, Alkylaryl- oder Cycloalkylrest sein. Weiterhin enthält das Phosphit der allgemeinen Formel (4) mindestens einen $ac^-$- Rest, also mindestens eine Sulfonat-($-SO_3^-$) oder Carboxylat ($-COO^-$)-gruppe.

Die sulfonierten oder carboxylierten Ester der phosphorigen Säure kann man durch Umesterung (Alkoholyse) von Phosphorigsäureestem mit dem Salz, vorzugsweise dem Ammoniumsalz, einer Hydroxysulfonsäure oder einer Hydroxycarbonsäure erhalten. Hierzu wird das in einem organischen Lösungsmittel gelöste Salz bei 20 bis 200°C, vorzugsweise 80 bis 160°C, mit dem Phosphorigsäureester umgesetzt. Die Reaktanten werden üblicherweise in äquivalenten Mengen verwendet, wenngleich es auch möglich ist, einen der beiden Reaktionspartner im Überschuß einzusetzen. Die Reaktion wird durch Katalysatoren wie Amine, Natrium, Natriumalkoholate, Aluminiumtrichlorid, Titansäureester oder Phosphorigsäuredialkylester beschleunigt. Zur Umesterung geeignete Phosphorigsäureester leiten sich von aliphatischen oder aromatischen Hydroxyverbindungen ab, vorzugsweise solchen, die 1 bis 12 Kohlenstoffatomen enthalten. Beispiele für Phosphorigsäureester sind Trimethylphosphit, Triethylphosphit; Butyldiethylphosphit, Tri-n-propylphosphit, Tri-n-butylphosphit, Tri-2-ethylhexylphosphit, Tri-n-octylphosphit, Tri-n-dodecylphosphit, Dimethylphenylphosphit, Diethylphenylphosphit, Triphenylphospit. Bevorzugtes organisches Phosphit ist das Triphenylphosphit.

Als Kationen $Q^+$ enthalten die erfindungsgemäßen nichtwäßrigen ionischen Flüssigkeiten durch organische Reste substitutierte Ammoniumionen, nämlich Ammoniumionen, die sich von Mono- oder Diaminen ableiten. Die Ammoniumionen von Monoaminen entsprechen der allgemeinen Formel (5)

$$R^3R^4N^{\oplus} = CR^5R^6$$

(5)

wobei $R^3$, $R^4$, $R^5$ und $R^6$ gleich oder verschieden sind und Wasserstoff, insbesondere mit der Maßgabe, daß mindestens ein $R^3$, $R^4$, $R^5$, $R^6$ nicht Wasserstoff ist, oder einen linearen oder verzweigten, aliphatischen Kohlenwasserstoffrest mit 1 bis 20 Kohlenstoffatomen, einen cycloaliphatischen oder aromatischen Kohlenwasserstoffrest mit 6 bis 20 kohlenstoffatomen oder einen Alkoxyrest mit 1 bis 10 Kohlenstoffatomen bedeuten. Beispiele für solche Reste sind Alkyl-, Alkenyl-, Cycloalkyl-, Aryl- Alkylaryl-, oder Aralkylreste.

Als Kationen der erfindungsgemäßen nichtwäßrigen ionischen Flüssigkeiten kommen ferner Ionen in Betracht, die sich von ungesättigten cyclischen Verbindungen sowie von aromatischen Verbindungen mit jeweils einem dreibindigen N-Atom im 4- bis 10-, vorzugsweise 5- bis 6-gliedrigen heterocyclischen Ring ableiten. Solche Kationen lassen sich vereinfacht (d.h. ohne Angabe von genauer Lage und Anzahl der Doppelbindungen im Molekül) durch die nachstehende allgemeine Formel (6) wiedergeben.

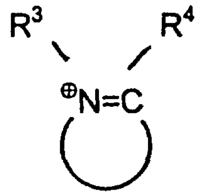

(6)

$R^3$ und $R^4$ besitzen dabei die vorgenannte Bedeutung. Beispiele für cyclische Amine der vorgenannten Art sind Pyridin, die isomeren Picoline und Lutidine, Chinolin und i-Chinolin.

Weitere Kationen gehen auf aliphatische, cycloaliphatische oder aromatische Diamine zurück. Sie folgen den allgemeinen Formeln (7) und (8)

$$R^3R^4R^5N^{\oplus}\text{-G-}N^{\oplus}R^6R^7R^8 \qquad (7)$$

$$R^3R^4N^{\oplus} = CR^5\text{-G-}R^5C = N^{\oplus}R^3R^4 \qquad (8)$$

in denen $R^3$, $R^4$, $R^5$, $R^6$, $R^7$ und $R^8$ gleich oder verschieden sind und Wasserstoff, einen linearen oder verzweigten aliphatischen Kohlenwasserstoffrest mit 1 bis 20 Kohlenstoffatomen, einen cycloaliphatischen oder aromatischen Kohlenwasserstoffrest mit 6 bis 30 Kohlenstoffatomen, einen Alkylarylrest mit 7 bis 40 Kohlenstoffatomen oder einen Alkoxyrest mit 1 bis 10 Kohlenstoffatomen bedeuten. G steht für einen Alkylenrest $(\text{-CHR}^9\text{-})_d$, wobei $R^9$ Wasserstoff oder ein Kohlenwasserstoffrest mit 1 bis 5 Kohlenstoffatomen und d eine ganze Zahl von 1 bis 8, vorzugsweise 2 bis 6 ist, für einen Arylenrest mit 6 bis 30 Kohlenstoffatomen oder für einen Alkylenarylrest mit 7 bis 40 Kohlenstoffatomen. Beispiele für die durch $R^3$, $R^4$, $R^5$, $R^6$, $R^7$ und $R^8$ bezeichneten Kohlenwasserstoffreste sind Alkyl-, Alkenyl-, Cycloalkyl-, Aryl, Alkylaryl-, oder Arylalkylreste, wie Methyl, Ethyl, Propyl, i-Propyl, Butyl, sek.-Butyl, t-Butyl, Amyl, Methylen, Ethyliden, Phenyl, Benzyl. $R^9$ wird beispielhaft durch den Methyl-, Ethyl-, n-Propyl-, i-Propylrest und die isomeren Butylreste beschrieben. Beispiele für G sind die Reste Methylen, Ethylen, Propylen, Butylen, 1,4-Phenylen, 1,4-Tolylen, 1,4-Xylylen, 1,1'-Biphenyl-4,4'-diyl, 1,4-Naphthylen, 1,1-Binaphthyl-2,2'-diyl.

Besonders geeignete Kationen der erfindungsgemäßen nichtwäßrigen ionischen Flüssigkeiten leiten sich von 1-Amino-3-dialkylaminopropanen der allgemeinen Formel (9)

$$R^{10}R^{11}N\text{-}CH_2\text{-}CH_2\text{-}CH_2\text{-}NH_2 \qquad (9)$$

als Diaminen ab, in der $R^{10}$ und $R^{11}$ gleiche oder verschiedene lineare oder verzweigte Alkylreste mit 4 bis 20 Kohlenstoffatomen sind und beispielsweise n-Butyl, n-Pentyl, n-Hexyl, n-Heptyl, i-Heptyl, n-Octyl, i-Octyl, n-Nonyl, i-Nonyl, n-Decyl, i-Decyl, n-Undecyl, i-Undecyl, n-Dodecyl oder i-Dodecyl bedeuten.

Weitere vorteilhafte Kationen der erfindungsgemäßen nichtwässrigen ionischen Flüssigkeiten gehen auf folgende Amine zurück: 1-Amino-3-(di-nheptyl)-aminopropan, 1-Amino-3-(di-i-heptyl)-aminopropan, 1-Amino-3-(di-noctyl)-aminopropan, 1-Amino-3-(di-i-octyl)-aminopropan, 1-Amino-3-(di-nnonyl)-aminopropan, 1-Amino-3-(di-i-nonyl)-aminopropan, 1-Amino-3-(di-nundecyl)-aminopropan, 1-Amino-3-(di-i-undecyl)-aminopropan, 1-Amino-3-(di-n-dodecyl)-aminopropan oder 1-Amino-3-(di-i-dodecyl)-aminopropan.

Die vorstehend beschriebenen 1-Amino-3-dialkylaminopropane sind leicht aus N,N-(dialkyl)aminen und Acrylnitril zugänglich (vgl. Ullmanns Encyclopedia of Industrial Chemistry, Vol. A2, 1985).

Schließlich zählen zu den Diaminen, die geeignete Kationen für die erfindungsgemäßen, nichtwäßrigen ionischen Flüssigkeiten ergeben, auch heterocyclische Verbindungen. Zu ihnen zählen gesättigte oder ungesättigte sowie aromatische Verbindungen mit jeweils zwei dreibindigen N-Atomen im 4- bis 10-, vorzugsweise 5- oder 6-gliedrigen heterocyclischen Ring. Diese Verbindungen können sowohl an den Kohlenstoffatomen, als auch an den Stickstoffatomen substituiert sein, vorzugsweise durch Alkylreste mit 1 bis 10 Kohlenstoffatomen und durch Phenylreste. Sie können weiterhin durch, gegebenenfalls substituierte, Benzolringe und/oder Cyclohexanringe unter Ausbildung mehrkerniger Strukturen anelliert sein. Beispiele für solche Verbindungen sind Pyrazol, 3,5-Dimethylpyrazol, Imidazol, Benzimidazol, Dihydropyrazol, Pyrazolidin, Pyridazin, Pyrimidin, Pyrazin, 2,3-, 2,5- und 2,6-Dimethylpyrazin, Cimolin, Phthalazin, Chinazolin, Phenazin und Piperazin. Vom Imidazol und seinen Alkyl- und Phenylderivaten abgeleitete Kationen der allgemeinen Formel (10)

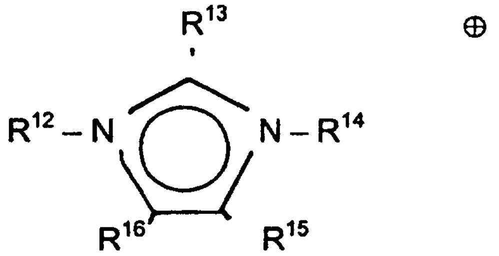

(10)

haben sich als Bestandteil der neuen ionischen Flüssigkeiten bewährt. In dieser Formel sind $R^{12}$, $R^{13}$, $R^{14}$, $R^{15}$ und $R^{16}$ gleich oder verschieden. Sie stehen für Wasserstoff, einen $C_1$- bis $C_{30}$-Alkylrest, einen $C_6$- bis $C_{40}$-Arylrest, einen $C_7$- bis $C_{40}$-Alkylarylrest oder einen $SiR_3{}^{17}$-rest, in dem $R^{17}$ einen $C_1$-bis $C_{30}$-Alkylrest oder einen $C_6$- bis $C_{40}$-Arylrest bedeutet. Beispiele für solche Kationen sind: 1-Ethyl-3-methyl-2,4,5-H-imidazolium, 1-Propyl-3-methyl-2,4,5-H-imidazolium, 1-Butyl-3-methyl-2,4,5-H-imidazolium, 1,3,4,5-Tetramethyl-2-H-imidazolium, 2,4,5-Trimethyl-1,3-H-imidazolium, 1,2,3,4,5-Pentamethylimidazolium, 1,2,3,5-Tetramethyl-4-H-imidazolium, 1,2,3,4-Tetramethyl-5-H-imidazolium, 1,3,4,5-Tetraphenyl-2-H-imidazolium, 1,3-Dimethyl-4,5-diphenyl-2-H-imidazolium, 1-Ethyl-3-isopropyl-2,4,5-H-imidazolium, 1-Butyl-3-octanyl-2,4,5-H-imidazolium, 1-Propyl-3-octanyl-2,4,5-H-imidazolium, 1-Ethyl-3-octanyl-2,4,5-H-imidazolium, 1-Methyl-3-octanyl-2,4,5-Himidazolium, 1,3-Diisoproypl-4,5-dimethyl-2-H-imidazolium, 1,4,5-Trimethyl-3-trimethylsilyl-2-H-imidazolium, 2-Ethyl-4-methyl-1,3,5-H-imidazolium, 1,3-Adamantyl-4,5-dimethyl-1-H-imidazolium, 1,2,4,5-Tetramethyl-3-H-imidazolium, 1-Methyl-2,3,4,5-H-imidazolium, 1,3-Dimethyl-2,4,5-H-imidazolium, 2-Methyl-4,5-ethyl-1,3-H-imidazolium, 2,4,5-Trimethyl-1,3-H-imidazolium, 1-Ethyl-2,3,4,5-H-imidazolium, 1,3-Diethyl-4,5-dimethyl-2-H-imidazolium, 1,3-Diphenyl-4,5-dimethyl-2-H-imidazolium, 1,3-Diphenyl-2,4,5-H-imidazolium, 1,3-Dimethoxy-4,5-dimethyl-2-H-imidazolium, 1-Trimethylsilyl-2,3,5-trimethyl-4-H-imidazolium.

Weiterhin haben sich ionische Flüssigkeiten auf Basis sulfonierter oder carboxylierter Triester der phosphorigen Säure sehr bewährt, deren Kationen sich von Polyaminen ableiten. Beispiele für solche Polyamine sind Hexamethylentetramin und Purin sowie dessen Derivate.

Zur Herstellung der erfindungsgemäßen nichtwäßrigen ionischen Flüssigkeiten geht man von Salzen der weiter oben ausführlich beschriebenen sulfonierten oder carboxylierten Ester der phosphorigen Säure aus. Geeignet sind Salze der Alkali- und Erdalkalimetalle, vorzugsweise die Natrium- oder Kaliumsalze. Sie werden als wäßrige Lösungen der reinen Verbindungen oder aber auch als Mischung verschiedener Salze eingesetzt.

Zur Gewinnung der efindungsgemäßen nichtwäßrigen ionischen Flüssigkeiten wird das Amin zu dem einfach- oder mehrfachgeladenen Kation mit Säuren und/oder Alkylierungsmitteln in Gegenwart einer wäßrigen Lösung von Salzen der sulfonierten oder carboxylierten Phosphorigsäureester protoniert oder alkyliert.

Als Säuren können Wasserstoffsäuren, z.B. Tetrafluoroborsäure oder Hexafluorophosphorsäure oder Sauerstoffsäuren, z.B. Phosphorsäure, Schwefelsäure, Salpetersäure, ferner Phosphonsäuren mit 1 bis 20 Kohlenstoffatomen oder Sulfonsäuren mit 1 bis 20 Kohlenstoffatomen verwendet werden. Vorzugsweise setzt man wäßrige Schwefelsäure- oder Phosphorsäurelösungen ein, die im allgemeinen 10 bis 30 Gew.-%ige Säure enthalten.

Als Alkylierungsmittel verwendet man z.B. Mono- oder Dialkylsulfate oder Dialkylcarbonate mit 1 bis 41 Kohlenstoffatomen oder Alkylhalogenide mit 1 bis 10 Kohlenstoffatomen.

Säure und/oder Alkylierungsmittel setzt man üblicherweise in einer Menge von 0,9 bis 2,0, vorzugsweise 1,0 bis 1,5 Äquivalenten je Äquivalent der verwendeten Amine zu. Bei Verwendung einer Säure beträgt der pH-Wert nach Säurezugabe 2 bis 5, vorzugsweise 3 bis 4.

Zur Substitution der Metallionen in den Salzen der Phosphorigsäure-Ester durch Ammoniumionen setzt man die Amine, bezogen auf die Metallionen, vorteilhaft im Überschuß über die stöchiometrisch erforderliche Menge ein. Dieser Überschuß beträgt im allgemeinen bis zu 5 Äquivalenten, vorzugsweise bis zu 1 Äquivalent.

Das Amin wird üblicherweise als 20 bis 70 Gew.-%ige, vorzugsweise 40 bis 60 Gew.-%ige Lösung in einem organischen Lösungsmittel verwendet. Als organische Lösungsmittel sind aliphatische oder aromatische Kohlenwasserstoffe, wie Benzol, Toluol, o-Xylol, m-Xylol, p-Xylol, Mesitylen, n-Heptan, n-Octan, Cyclohexan oder auch Ether, z. B. 1,4-Dioxan oder Tetrahydrofuran geeignet. Vorzugsweise verwendet man Toluol oder Cyclohexan.

Die Zugabe der Säure und/oder des Alkylierungsmittels zu der Mischung aus der wäßrigen Lösung des oder der Salze des Phosphorigsäure-Esters und der organischen Lösung des Amins erfolgt bei 0 bis 60°C, vorzugsweise 20 bis 30°C. Die Dauer der Zugabe liegt im allgemeinen zwischen 0,5 bis 3 Stunden, vorzugsweise zwischen 1 und 2 Stunden.

Als Ergebnis der Umsetzung erhält man drei Phasen, eine untere wäßrige Phase, die das aus den Estern der phosphorigen Säure freigesetzte Alkaliund/oder Erdalkalisalz gelöst enthält, eine mittlere Phase, nämlich die nichtwäßrige ionische Flüssigkeit und eine obere Phase, die aus dem organischen Lösungsmittel, das gegebenenfalls

überschüssiges Amin enthält, besteht. Die gewünschte nichtwäßrige ionische Flüssigkeit läßt sich durch einfache Phasentrennung gewinnen.

Um eine einwandfreie Ausbildung der drei Phasen sicherzustellen, kann es sich als zweckmäßig erweisen, nach Zugabe der Säure und/oder des Alkylierungsmittels dem Gemisch weiteres organisches Lösungsmittel hinzuzusetzen. Vorzugsweise verwendet man das gleiche organische Lösungsmittel wie zum Lösen des Amins. Die Menge des zugesetzten organischen Lösungsmittels, die erforderlich ist, um eine Trennung in drei Phasen zu erreichen, kann durch einfache Vorversuche ermittelt werden.

In einer weiteren Ausführungsform des Herstellungsverfahrens kann zunächst eine wäßrige Lösung von Salzen der Phosphorigsäure-Ester mit einer Säure und/oder mit einem Alkylierungsmittel versetzt und anschließend das Amin, gelöst in einem organischen Lösungsmittel, zugegeben werden. Es ist auch möglich, das zu protonierende und/oder zu alkylierende Amin zunächst mit der Säure und/oder dem Alkylierungsmittel umzusetzen und anschließend eine wäßrige Lösung der Salze der sulfonierten oder carboxylierten Ester der phosphorigen Säure hinzuzugeben.

Schließlich ist es auch möglich, die Salze der sulfonierten oder carboxylierten Phosphorigsäure-Ester durch Behandlung mit einem Kationenaustauscher in der $H^+$-Form in die freie Sulfonsäure bzw. Carbonsäure zu überführen und diese Säure daraufhin mit dem Amin zu neutralisieren.

Die zweite Komponente des Katalysatorsystems, das Rhodium, kann entweder als Metall in feinverteilter Form, vorzugsweise auf einem Träger wie Aktivkohle, Calciumcarbonat, Tonerde oder ähnlichen Substraten oder als Rhodiumverbindung eingesetzt werden. Beispiele für anorganische oder organische Rhodiumverbindungen, in denen das Rhodium in seinen verschiedenen Oxidationsstufen vorliegen kann, sind die Rhodiumoxide $Rh_2O$, $Rh_2O_3$, $RhO_2$, $RhO_3$, die Salze der anorganischen Wasserstoffsäuren wie Halogenide, Sulfide, Selenide und Telluride, die Salze anorganischer Sauerstoffsäuren wie Rhodiumnitrat, Rhodiumsulfat, Rhodiumperchlorat, sowie die Salze aliphatischer Mono- oder Polycarbonsäuren wie Rhodiumacetat, Rhodiumpropionat, Rhodiumoxalat, Rhodiummalonat und Rhodium-2-ethylhexanoat. Weiterhin haben sich Carbonylverbindungen des Rhodiums wie Tricarbonylrhodium, $Rh(CO)_3$, Tetracarbonylrhodium, $[Rh(CO)_4]_2$, Tetrarhodiumdodekacarbonyl, $Rh_4(CO)_{12}$ sehr bewährt. Halogencarbonylverbindungen wie Dicarbonylrhodiumbromid, $[Rh(CO)_2]Br$ und Dicarbonylrhodiumjodid, $[Rh(CO)_2]I$, können zwar auch eingesetzt werden, finden wegen des korrosiven Verhaltens der Halogenionen jedoch nur begrenzte Anwendung. Schließlich sind auch komplexe Verbindungen des Rhodiums, insbesondere Rhodium(III)-Verbindungen, geeignete Ausgangsmaterialien zur Herstellung der katalytisch aktiven Metallkomponente im Katalysatorsystem. Diese Verbindungen enthalten ein-, zwei- oder dreibindige Liganden wie β-Diketone, z.B. Acetylaceton, ferner Alkylamine, Alkyl- oder Aryldiamine, stickstoffhaltige Heterocyclen wie Pyridin oder aliphatische oder cycloaliphatische und diethylenisch ungesättigte Kohlenwasserstoffe wie Cyclopentadien und 1,5-Cyclooctadien. Für die Bildung des Katalysatorsystems besonders geeignete Rhodiumverbindugnen sind die Rhodiumoxide, die Rhodiumcarbonyle, Rhodiumacetat, Rhodium-2-ethylhexanoat und Rhodium(III)-acetylacetonat.

Das Katalysatorsystem kann im Anfangsstadium der Umsetzung in situ, d.h. in der Reaktionsphase, unter Reaktionsbedingungen und in Gegenwart des Olefins aus Ligandflüssigkeit und metallischem Rhodium oder einer Rhodiumverbindung hergestellt werden. Es ist aber auch möglich, das Katalysatorsystem getrennt von der Hydroformylierungsstufe in einem eigenen Reaktionsschritt zu präformieren und darauf dem Reaktionsgemisch zuzusetzen. Zur Präformierung suspendiert oder löst man metallisches Rhodium oder eine Rhodiumverbindung in der Ligandflüssigkeit und behandelt das Gemisch mit Synthesegas. Typische Reaktionsbedingungen sind Temperaturen von 90 bis 150°C, insbesondere 100 bis 120°C und Drücke von 0,2 bis 10, vorzugsweise 0,5 bis 5 MPa. Die Reaktionszeit beträgt in Abhängigkeit von den gewählten Reaktionsbedingungen bis zu 5 h. Die Gegenwart eines zusätzlichen Lösungsmittels während des Präformierungsschrittes ist möglich. Geeignet sind aliphatische oder aromatische Kohlenwasserstoffe wie Heptan, Cycloheptan, Toluol, o-Xylol, m-Xylol und p-Xylol. Das Verhältnis von Rhodium zu Ligandflüssigkeit (auch als P(III) : Rh-Molverhältnis bezeichnet) kann sich über einen sehr weiten Bereich erstrecken und 1000 : 1 und mehr betragen. Als untere Grenzen bewährt haben sich 3 bis 300 und insbesondere 20 bis 200 mol P(III) je mol Rh.

Die Konzentration des Rhodiums, bezogen auf eingesetztes Olefin, beträgt 2 bis 1000 Gew.-ppm, vorzugsweise 3 bis 400 Gew.-ppm und insbesondere 5 bis 100 Gew.-ppm.

Die Umsetzung der Olefine bzw. der olefinisch ungesättigten Verbindungen mit Wasserstoff und Kohlenmonoxid zu Carbonylverbindungen erfolgt bei Temperaturen von 20 bis 150°C, bevorzugt 80 bis 140°C und insbesondere 100 bis 125°C und Drücken von 0,1 bis 20 MPa, vorzugsweise 1 bis 12 MPa und insbesondere 3 bis 7 MPa. Die im Einzelfall anzuwendenden Reaktionsbedingungen hängen auch von der Art der umzusetzenden olefinischen Verbindung ab. So lassen sich reaktionsfähige Einsatzstoffe bereits bei relativ niedrigen Temperaturen und Drücken und in Gegenwart geringer Katalysatormengen umsetzen, während reaktionsträgere Verbindungen entsprechend energischere Reaktionsbedingungen erfordern.

Die Zusammensetzung des Synthesegases, d.h. die Anteile von Kohlenmonoxid und Wasserstoff im Gasgemisch, kann in weiten Grenzen variiert werden. Im allgemeinen setzt man Gemische ein, in denen das Volumenverhältnis von Kohlenmonoxid zu Wasserstoff 5 : 1 bis 1 : 5 beträgt. Üblicherweise ist dieses Verhältnis 1 : 1 oder weicht von diesem Wert nur wenig ab.

Die olefinische Verbindung kann als solche oder in Lösung der Hydroformylierung zugeführt werden. Geeignete Lösungsmittel sind Ketone wie Aceton, Methylethylketon, Acetophenon, niedere aliphatische Nitrile wie Acetonitil, Propionitril oder Benzonitril, Dimethylformamid, lineare oder verzweigte gesättigte aliphatische Monohydroxyverbindungen wie Methanol, Ethanol, Propanol und Isopropanol, aromatische Kohlenwasserstoffe wie Benzol oder Toluol und gesättigte cycloaliphatsiche Kohlenwasserstoffe wie Cyclopentan oder Cylcohexan.

Das erfindungsgemäße Verfahren kann sowohl absatzweise als auch kontinuierlich durchgeführt werden. Nach Beendigung der Umsetzung erhält man zwei Phasen, das spezifisch leichtere Reaktionsprodukt als obere und die spezifisch schwerere Katalysatorlösung als untere Phase. Beide Stoffgemische lassen sich in einfacher Weise, z. B. durch Dekantieren, voneinander trennen. Das Katalysatorsystem kann partiell oder vollständig in den Hydroformylierungsprozeß zurückgeführt, das Produkt der Weiterverarbeitung, z.B. einem Reinigungsprozeß oder einer nachfolgenden Umsetzung, zugeleitet werden.

Die Anwendung des neuen Verfahrens ist nicht auf bestimmte Olefine als Ausgangsstoffe beschränkt. Dementsprechend können aliphatische, cycloaliphatische oder araliphatische Verbindungen, die eine oder mehrere olefinische Doppelbindungen besitzen und gegebenenfalls auch noch funktionelle Gruppen enthalten, umgesetzt werden Beispiele für aliphatische Verbindungen sind lineare oder verzweigte Olefine mit end- oder innenständigen Doppelbindungen wie Ethylen, Propylen, Buten-1, Isobuten, Penten-1, 2-Methylbuten-1, Hexen-1, Hepten-1, Octen-1, Octen-3, 2,4,4-Trimethylpenten-1, Nonen-1, 2-Propylhexen-1, Decen-1, Decen-3, Undecen-3, 4,4-Methylnonen-1, 6-Propyldecen-1. Auch konjugierte Polyolefine, wie z.B. Butadien-1.3 lassen sich mit Erfolg umsetzen. Als cycloaliphatische Einsatzstoffe kommen z.B. Dicyclopentadien, Vinylcyclohexen, Cyclooctadien und cyclische Terpene wie Limonen, und Pinen in Betracht. Beispiele für araliphatsiche Olefine sind Styrol, $\alpha$-Methylstyrol, 1,1-Diphenylethylen, Divinylbenzol und m-Hexylstyrol.

Beispiele für olefinische Verbindungen mit funktionellen Gruppen sind Alkohole, Aldehyde, Carbonsäuren, Ester, Nitrile und Halogenverbindungen. Zu ihnen gehören Vinylverbindungen, insbesondere Ether und Ester wie Vinylmethylether, Vinylethylether, $\beta$-Vinylnaphthalin, o-Vinyl-p-xylol, Vinylacetat; Allylverbindungen, unter ihnen insbesondere die Alkohole und Ester wie Allylalkohol, Allyethylether und Allylacetat; Aldehyde wie Acrolein, Methacrolein, Crotonaldehyd; Ester der Acrylsäure, der Methacrylsäure, der Fumarsäure und der Maleinsäure; Acrylnitril. Diese Aufzählung geeigneter Ausgangsstoffe ist nicht erschöpfend, sondern lediglich beispielhaft.

Der erfindungsgemäße Prozeß eignet sich insbesondere zur Hydroformylierung wasserempfindlicher Olefine und Olefinderivate, wie die Ester des Vinylalkohols, z.B. Vinylacetat, Vinylpropionat, des Allylalkohols, wie Allylacetat, Allypropionat, Allylbutyrat, die Ester der Acrylsäure und die Acetale des Acroleins. Mit besonderem Erfolg lassen sich nach dem neuen Verfahren Olefine und Olefinderivate mit 2 bis 20 Kohlenstoffatomen hydroformylieren.

Die folgenden Beispiele sollen die Erfindung erläutern, diese aber nicht einschränken. Das in Beispiel eingesetzte Phosphit wurde analog Literaturangaben (EP 353770) hergestellt.

Beispiel 1:

In einem 1l Dreihalskolben mit Bodenablauf wurden bei Raumtemperatur 103,9g einer 4 Natriumsulfonatphenyl-(3,3' di-t-butyl-5,5'-dimethoxy-1,1'biphenyl-2,2'-diyl)phosphit (200 mmol) gelöst in 396g dest. Wasser mit einer Lösung von 65,3g 1-Amino-3-(di-i-nonyl)-aminopropan (200 mmol) in 400 ml Toluol langsam versetzt. Unter Rühren wird über einen Zeitraum von 2 Stunden 98g Schwefelsäure (20 Gew%ig) zugetropft. Nach dem Abstellen des Rührers bilden sich 3 Phasen aus, die jeweils abgetrennt und auf ihren P(III)-Gehalt analysiert wurden. Die gesamte P(III) Menge befindet sich in den 280g der mittleren Phase, welche die nichtwäßrige, ionogene Ligandflüssigkeit bildet. Die untere Phase enthält Natriumhydrogensulfat bzw. Natriumsulfat, während die obere Phase hauptsächlich aus Toluol besteht.

Beispiel 2:

Einem mit Rührer ausgerüsteten 0,21 Edelstahlautoklav werden Propylen und ein aus gleichen Volumenteilen bestehendes $CO/H_2$-Gemisch in einer solchen Menge zugeleitet, daß 10 Nl/h Abgas (1 Normliter ist gleich 1 Liter bei einem technischen Druck von 1 atm und einer Temperatur von 20°C). aus dem Reaktor entnommen werden können. Gleichzeitig werden je Stunde 280g nichtwäßrige, ionogene Ligandflüssigkeit im Kreis durch den Reaktor geführt. Rhodium wurde in Form vom $RhCl_3$ zugesetzt. Die Hydroformylierungen wurden halbkontinuierlich über 8 Stunden durchgeführt, wobei zwecks Reproduktion jeweils ein Doppelversuch durchgeführt wurde. Die übrigen Reaktionsparameter sind in der Tabelle zu entnehmen.

| Versuchsdauer [h] | 8 | 8 |
|---|---|---|
| **Temperatur [°C]** | 122 | 122 |

(fortgesetzt)

| | | |
|---|---|---|
| **Druck [bar]** | 50 | 50 |
| **Rh-Gehalt [mg/kg]** | 278 | 281 |
| **P(III) Gehalt [mmol/kg]** | 280 | 280 |
| Ligand/Rh | 100 | 99 |
| **C3-Einsatz [g/h]** | 40 | 40 |
| **Aktivität** | 15,6 | 16,01 |
| **Produktivität** | 0,222 | 0,225 |
| **Umsatz [%]** | 39 | 41 |
| **n/i-Verhältnis** | 97/3 | 97/3 |

**Patentansprüche**

**1.** Zweiphasiges Verfahren zur Herstellung von Aldehyden durch Umsetzung von Monoolefinen, konjugierten und nichtkonjugierten Polyolefinen, Cylcoolefinen oder Derivaten dieser Verbindungsklassen mit Kohlenmonoxid und Wasserstoff bei Temperaturen von 20 bis 150°C und Drücken von 0,1 bis 20 MPa, charakterisiert durch das Vorliegen einer organischen aldehydhaltigen Phase und einer darin nicht löslichen, nichtwäßrigen ionischen Flüssigkeit der allgemeinen Formel $(Q^+)_aA^{a-}$ mit mindestens einer Rhodiumverbindung, **dadurch gekennzeichnet, daß** $Q^+$ für von Monoaminen abgeleitete Ammoniumionen der allgemeinen Formel (5)

$$R^3R^4N^{\oplus} = CR^5R^6 \qquad (5)$$

steht, wobei $R^3$, $R^4$, $R^5$, $R^6$ gleich oder verschieden sind und Wasserstoff, insbesondere mit der Maßgabe bedeuten, daß mindestens ein $R^3$, $R^4$, $R^5$, $R^6$ nicht Wasserstoff ist, oder für einen linearen oder verzweigten aliphatischen Kohlenwasserstoffrest mit 1 bis 20 Kohienstoffatomen, einen cycloaliphatischen oder aromatischen Kohlenwasserstoffrest mit 6 bis 20 Kohlenstoffatomen oder einen Alkoxyrest mit 1 bis 10 Kohlenstoffatomen stehen; oder für von Monoaminen abgeleitete Ammoniumionen der allgemeinen Formel (6)

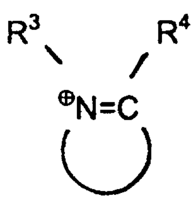

**(6)**

steht, die sich von gesättigten oder ungesättigten cyclischen Verbindungen sowie von aromatischen Verbindungen mit jeweils einem dreibindigen N-Atom im 4- bis 10-, vorzugsweise 5- bis 6-gliedrigen Ring ableiten, wobei $R^3$, $R^4$ gleich oder verschieden sind und Wasserstoff, einen linearen oder verzweigten aliphatischen Kohlenwasserstoffrest mit 1 bis 20 Kohlenstoffatomen, einen cycloaliphatischen oder aromatischen Kohlenwasserstoffrest mit 6 bis 20 Kohlenstoffatomen oder einen Alkoxyrest mit 1 bis 10 Kohlenstoffatomen bedeuten; oder für von Diaminen abgeleitete Ammoniumionen der allgemeinen Formel (7) oder (8)

$$R^3R^4R^5N^{\oplus}\text{-G-}N^{\oplus}R^6R^7R^8 \qquad (7)$$

$$R^3R^4N^{\oplus} = CR^5\text{-G-}R^5C = N^{\oplus}R^3R^4 \qquad (8)$$

steht, wobei $R^3$, $R^4$, $R^5$, $R^6$, $R^7$ und $R^8$ gleich oder verschieden sind und Wasserstoff, einen linearen oder verzweigten aliphatischen Kohlenwasserstoffrest mit 1 bis 20 Kohlenstoffatomen, einen cycloaliphatischen oder aromatischen Kohlenwasserstoffrest mit 6 bis 30 Kohlenstoffatomen, einen Alkylarylrest mit 7 bis 40 Kohlenstoffatomen oder einen Alkoxyrest mit 1 bis 10 Kohlenstoffatomen bedeuten, G für einen Alkylenrest ($-CHR^9-)_d$, wobei $R^9$ Wasserstoff oder ein Kohlenwasserstoffrest mit 1 bis 5 Kohlenstoffatomen und d eine ganze Zahl von 1 bis 8, vorzugsweise 2 bis 6 ist, für einen Arylenrest mit 6 bis 30 Kohlenstoffatomen oder einen Alkylenarylrest mit 7 bis 40 Kohlenstoffatomen, steht; oder für vom Imidazol und seinen Alkyl- und Phenylderivaten abgeleitete Kationen der allgemeinen Formel (10)

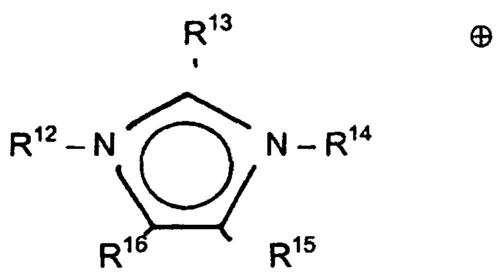

(10)

steht, in der $R^{12}$, $R^{13}$, $R^{14}$, $R^{15}$ und $R^{16}$ gleich oder verschieden sind und für Wasserstoff, einen $C_1$- bis $C_{30}$-Alkylrest, einen $C_6$- bis $C_{40}$-Arylrest, einen $C_7$- bis $C_{40}$-Alkylarylrest oder einen $SiR_3{}^{17}$-Rest, in dem $R^{17}$ einen $C_1$- bis $C_{30}$-Alkylrest oder einen $C_6$- bis $C_{40}$-Arylrest bedeutet, stehen, $A^{a-}$ für das Anion eines sulfonierten oder carboxylierten Triesters der phosphorigen Säure steht und a eine ganze Zahl und mindestens gleich 1 ist und dass die Aldehyd enthaltende Produktphase und die Rhodium und ionische Flüssigkeit enthaltende Katalysatorphase durch Phasentrennung voneinander getrennt werden und die Katalysatorphase partiell oder vollständig in den Hydroformylierungsprozeß zurückgeführt wird.

2. Verfahren nach Anspruch 1, **dadurch gekennzeichnet, daß** die Alkoholkomponente des Phosphorigsäuretriesters der allgemeinen Formel

$$(Qac)_b\text{-}Y\text{-}(OH)_c \quad (1)$$

entspricht, in der Y ein organischer Rest ist, Q die in Anspruch 1 definierte Bedeutung hat, ac für einen Sulfonsäure- oder Carbonsäurerest steht und b und c ganze Zahlen sind und jeweils mindestens den Wert 1, c insbesondere den Wert 1 oder 2 haben.

3. Verfahren nach Anspruch 2, **dadurch gekennzeichnet, daß** Y ein linearer oder verzweigter, gesättigter aliphatischer Rest mit 1 bis 20 Kohlenstoffatomen ist, der durch Hydroxy- oder Alkoxyreste mit 1 bis 10 Kohlenstoffatomen substituiert sein kann, ein gesättigter oder ungesättigter, ein- oder mehrkerniger cycloaliphatischer Rest mit 5 bis 14 Kohlenstoffatomen im Ring oder den Ringen, ein ein- oder mehrkemiger aromatischer Rest mit 6 bis 14 Kohlenstoffatomen im Ring oder den Ringen ist, wobei der cycloaliphatische Rest und der aromatische Rest jeweils durch Alkylreste mit 1 bis 20 Kohlenstoffatomen, durch Aryl-, Alkylaryloder Aralkylreste mit 6 bis 30 Kohlenstoffatomen, durch Cycloalkylreste mit 5 bis 14 Kohlenstoffatomen, durch Hydroxygruppen und durch Alkoxyreste mit 1 bis 10 Kohlenstoffatomen substituiert sein können oder ein gesättigter oder ungesättigter heterocyclischer Rest mit einem Heteroatom oder mehreren Heteroatomen aus der Gruppe N, O, S im Molekül ist, der gegebenenfalls durch Alkylreste oder Arylreste substituiert oder mit cycloaliphatischen oder aromatischen Ringsystemen kondensiert ist.

4. Verfahren nach Anspruch 3, **dadurch gekennzeichnet, daß** Y ein vom Benzol, Toluol, Ethylbenzol, den isomeren Xylolen, von Biphenyl, Naphthalin, Binaphthyl, Benzyl oder Pyridin abgeleiteter Rest ist.

5. Verfahren nach den Ansprüchen 1 bis 4, **dadurch gekennzeichnet, daß** die Phosphorigsäuretriester der allgemeinen Formel

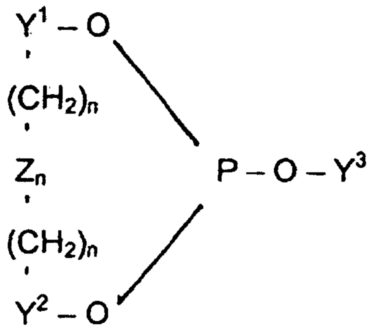

(2)

entsprechen, in der $Y^1$, $Y^2$ und $Y^3$ gleich oder verschieden sind und einen organischen Rest insbesondere einen vom Benzol, vom Naphthalin, vom Biphenyl oder vom Binaphthyl abgeleiteten Rest bedeuten, der jeweils durch einen oder mehrere Alkylreste mit 1 bis 20 Kohlenstoffatomen, durch einen oder mehrere Aryl-, Aralkyl-, Alkylarylreste mit 6 bis 30 Kohlenstoffatomen und/oder durch einen oder mehrere Cycloalkylreste mit 5 bis 14 Kohlenstoffatomen, durch Hydroxygruppen und/oder Alkoxyreste mit 1 bis 10 Kohlenstoffatomen und/oder Säurereste (-$ac^-$) substituiert sein kann, Z für eine zweiwertige Brückengruppe, nämlich für -$CR^1R^2$-, wobei $R^1$ und $R^2$ unabhängig voneinander Wasserstoff oder Alkylreste mit 1 bis 12 Kohlenstoffatomen sind, für -O-, -S-, -CO-, -$CH_2$-CO-$CH_2$-, insbesondere für -$CH_2$-, - O-, -CO-, und -$CH_2$-CO-$CH_2$- steht und n gleich oder verschieden und 0 oder 1 und im Falle Z gleich -$CR^1R^2$- 1, 2 oder 3 ist und der Ester gemäß der Formel (2) mindestens einen Säurerest (-$ac^-$) enthält.

6. Verfahren nach den Ansprüchen 1 bis 4, **dadurch gekennzeichnet, daß** die Phosphorigsäuretriester der allgemeinen Formel

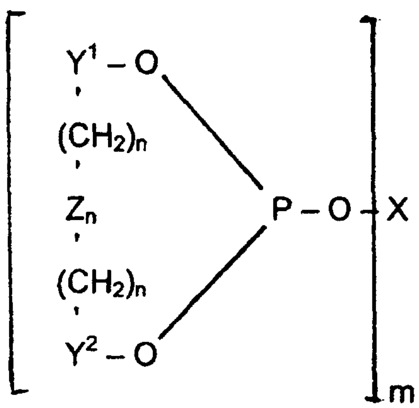

(3)

entsprechen, in der $Y^1$ und $Y^2$ gleich oder verschieden sind und einen organischen Rest, insbesondere einen vom Benzol, vom Naphthalin, vom Biphenyl oder vom Binaphthyl abgeleiteten Rest bedeuten, der jeweils durch einen oder mehrere Alkylreste mit 1 bis 20 Kohlenstoffatomen, durch einen oder mehrere Aryl-, Aralkyl-, Alkylarylreste mit 6 bis 30 Kohlenstoffatomen und/oder durch einen oder mehrere Cycloalkylreste mit 5 bis 14 Kohlenstoffatomen, durch Hydroxygruppen und/oder Alkoxyreste mit 1 bis 10 Kohlenstoffatomen und/oder Säurereste (-$ac^-$) substituiert sein kann, Z für eine zweiwertige Brückengruppe, nämlich für -$CR^1R^2$-, wobei $R^1$ und $R^2$ unabhängig voneinander Wasserstoff oder Alkylreste mit 1 bis 12 Kohlenstoffatomen sind, für -O-, -S-, -CO-, - $CH_2$-CO-$CH_2$-, insbesondere für -$CH_2$-, -O-, -CO- und -$CH_2$-CO-$CH_2$-steht, n gleich oder verschieden und 0 oder 1 und im Falle Z gleich - $CR^1R^2$- 1, 2 oder 3 ist, X eine m-wertige Brückengruppe aus der Gruppe der Alkylenreste, Alkylenoxyalkylenreste, Arylenreste oder Aryl-$Z_n$-arylreste bedeutet, insbesondere für einen Alkylenrest mit 2 bis 18, vorzugsweise 2 bis 12 Kohlenstoffatomen und in der Bedeutung Aryl-$Z_n$-aryl Z für -$CH_2$-CO-$CH_2$- steht, wobei die durch X bezeichneten Reste durch einen oder mehrere Alkyl- und/oder Alkoxyreste und/oder durch einen oder mehrere Säurereste (-$ac^-$) substituiert sein können und der Ester gemäß der Formel (3) mindestens einen Säurerest (-$ac^-$) enthält und m eine ganze Zahl von 2 bis 6 ist.

7. Verfahren nach den Ansprüchen 1 bis 4, **dadurch gekennzeichnet, daß** die Phosphorigsäuretriester der allgemeinen Formel

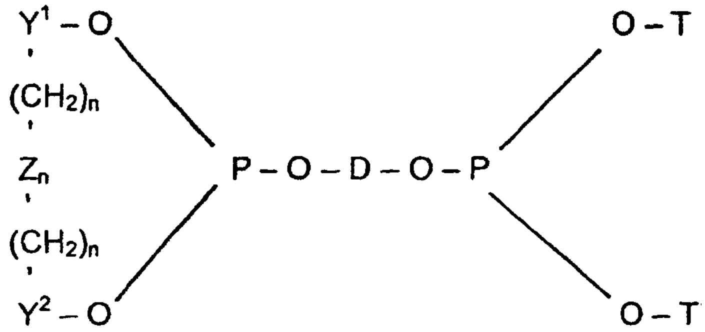

(4)

entsprechen, in der $Y^1$ und $Y^2$ gleich oder verschieden sind und einen organischen Rest, insbesondere einen vom Benzol, vom Naphthalin, vom Biphenyl oder vom Binaphthyl abgeleiteten Rest bedeuten, der jeweils durch einen oder mehrere Alkylreste mit 1 bis 20 Kohlenstoffatomen, durch einen oder mehrere Aryl-, Aralkyl-, Alkylarylreste mit 6 bis 30 Kohlenstoffatomen und/oder durch einen oder mehrere Cycloalkylreste mit 5 bis 14 Kohlenstoffatomen, durch Hydroxygruppen und/oder Alkoxyreste mit 1 bis 10 Kohlenstoffatomen und/oder Säurereste ($-ac^-$) substituiert sein kann, Z für eine zweiwertige Brückengruppe, nämlich für $-CR^1R^2-$, wobei $R^1$ und $R^2$ unabhängig voneinander Wasserstoff oder Alkylreste mit 1 bis 12 Kohlenstoffatomen sind, für -O-, -S-, -CO-, $-CH_2-CO-CH_2-$, insbesondere für $-CH_2-$, -O-, -CO-und $-CH_2-CO-CH_2-$ steht, n gleich oder verschieden ist und 0 oder 1 und im Falle $-CR^1R^2-$ 1, 2 oder 3 ist, D einen zweiwertigen Kohlenwasserstoffrest als Brückengruppe, nämlich einen Alkylenrest mit 1 bis 30 Kohlenstoffatomen, einen Arylen-, Alkylarylen-, Arylalkylenrest mit 6 bis 30 Kohlenstoffatomen und einen Aryl-$Z_n$-aryl-Rest bedeutet und T einen einwertigen Kohlenwasserstoffrest mit 1 bis 30 Kohlenstoffatomen wiedergibt und der Ester gemäß Formel (4) mindestens einen Säurerest ($-ac^-$) enthält.

8. Verfahren nach den Ansprüchen 1 bis 7, **dadurch gekennzeichnet, daß** je mol Rhodium mindestens 3 bis 300, vorzugsweise 2 bis 200 mol Phosphor(III) eingesetzt werden.

9. Verfahren nach den Ansprüchen 1 bis 8, **dadurch gekennzeichnet, daß** die Rhodium-Konzentration, bezogen auf eingesetztes Olefin, 2 bis 1000 Gew.-ppm, vorzugsweise 3 bis 400 Gew.-ppm und insbesondere 5 bis 100 Gew.-ppm beträgt.

10. Verfahren nach den Ansprüchen 1 bis 9, **dadurch gekennzeichnet, daß** die Umsetzung bei Temperaturen von 20 bis 150°C, vorzugsweise bei 80 bis 140°C und insbesondere bei 100 bis 125°C erfolgt.

11. Verfahren nach den Ansprüchen 1 bis 10, **dadurch gekennzeichnet, daß** die Umsetzung bei Drücken von 0,1 bis 20 MPa, vorzugsweise 1 bis 12 MPa und insbesondere 3 bis 7 MPa erfolgt.

12. Verfahren nach den Ansprüchen 1 bis 11, **dadurch gekennzeichnet, daß** Olefine und Olefinderivate mit 2 bis 20 Kohlenstoffatomen umgesetzt werden.

**Claims**

1. A two-phase process for preparing aldehydes by reacting monoolefins, conjugated and nonconjugated polyolefins, cycloolefins or derivatives of these classes of compound with carbon monoxide and hydrogen at temperatures of from 20 to 150°C and pressures of from 0.1 to 20 MPa, **characterized by** the presence of an organic aldehyde-containing phase and a nonaqueous ionic liquid of the formula $(Q^+)_aA^{a-}$, nonsoluble therein with at least one rhodium compound, wherein $Q^+$ means ammonium ions derived from monoamines and having the formula (5)

$$R^3R^4N^{\oplus} = CR^5R^6 \qquad (5)$$

where $R^3$, $R^4$, $R^5$, $R^6$ are identical or different and are each hydrogen, in particular with the proviso that at least one of $R^3$, $R^4$, $R^5$, $R^6$ is not hydrogen, or a linear or branched, aliphatic hydrocarbon radical having from 1 to 20 carbon atoms, a cycloaliphatic or aromatic hydrocarbon radical having from 6 to 20 carbon atoms or an alkoxy radical having from 1 to 10 carbon atoms; or means ammonium ions derived from monoamines and having the formula (6)

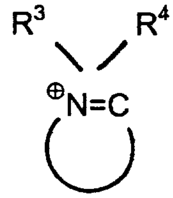

(6)

which are derived from saturated or unsaturated cyclic compounds or from aromatic compounds having a trivalent N atom in a 4- to 10-membered, preferably 5- to 6-membered ring, where $R^3$, $R^4$ are identical or different and are each hydrogen, a linear or branched aliphatic hydrocarbon radical having from 1 to 20 carbon atoms, a cycloaliphatic or aromatic hydrocarbon radical having from 6 to 20 carbon atoms or an alkoxy radical having from 1 to 10 carbon atoms; or means ammonium ions derived from diamines and having the formula (7) or (8)

$$R^3R^4R^5N^{\oplus}\text{-G-}N^{\oplus}R^6R^7R^8 \qquad (7)$$

$$R^3R^4N^{\oplus} = CR^5\text{-G-}R^5C = N^{\oplus}R^3R^4 \qquad (8)$$

where $R^3$, $R^4$, $R^5$, $R^6$, $R^7$ and $R^8$ are identical or different and are each hydrogen, a linear or branched aliphatic hydrocarbon radical having from 1 to 20 carbon atoms, a cycloaliphatic or aromatic hydrocarbon radical having from 6 to 30 carbon atoms, an alkylaryl radical having from 7 to 40 carbon atoms or an alkoxy radical having from 1 to 10 carbon atoms, G is an alkylene radical $(\text{-CHR}^9\text{-})_d$, where $R^9$ is hydrogen or a hydrocarbon radical having from 1 to 5 carbon atoms and d is an integer from 1 to 8, preferably from 2 to 6, an arylene radical having from 6 to 30 carbon atoms or an alkylenearyl radical having from 7 to 40 carbon atoms; or means cations derived from imidazole and its alkyl-and phenyl derivatives of the fomula (10)

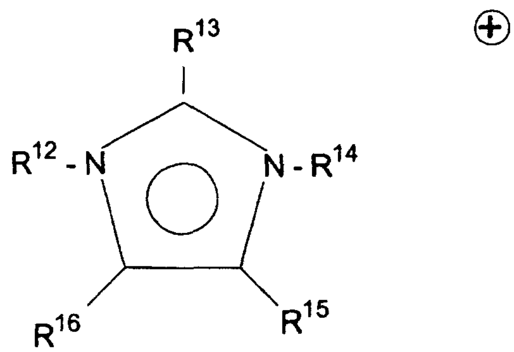

(10)

where $R^{12}$, $R^{13}$, $R^{14}$, $R^{15}$ and $R^{16}$ are identical or different and are each hydrogen, a $C_1$- to $C_{30}$-alkyl radical, a $C_6$- to $C_{40}$- aryl radical, a $C_7$- to $C_{40}$-alkyl aryl radical or a $SiR_3{}^{17}$ radical, in which $R^{17}$ means a $C_1$- to $C_{30}$-alkyl radical or a $C_6$- to $C_{40}$-aryl radical, $A^{a-}$ is the anion of a sulfonated or carboxylated triester of phosphorous acid and a is an integer equal to or greater than 1 and wherein the aldehyde-containing product phase and the catalyst phase comprising rhodium and ionic liquid are separated from one another by phase separation and the catalyst phase is partly or wholly returned to the hydroformylation process.

**2.** The process as claimed in claim 1, wherein the alcohol component of the phosphorous triester corresponds to the

formula

$$(Qac)_b\text{-}Y\text{-}(OH)_c \qquad (1)$$

where Y is an organic radical, Q has the meaning as defined in claim 1, ac is a sulfonic acid or carboxylic acid radical and b and c are integers which are each equal to or greater than 1, and c is particularly preferably 1 or 2.

**3.** The process as claimed in claim 2, wherein Y is a linear or branched, saturated aliphatic radical having from 1 to 20 carbon atoms which may be substituted by hydroxy groups or alkoxy radicals having from 1 to 10 carbon atoms, a saturated or unsaturated, monocyclic or polycyclic cycloalipahtic radical having from 5 to 14 carbon atoms in the ring or rings, a monocyclic or polycyclic aromatic radical having from 6 to 14 carbon atoms in the ring or rings, where the cycloaliphatic radical and the aromatic radical may each be substituted by alkyl radicals having from 1 to 20 carbon atoms, by aryl, alkylaryl or aralkyl radicals having from 6 to 30 carbon atoms, by cycloalkyl radicals having from 5 to 14 carbon atoms, by hydroxy groups and by alkoxy radicals having from 1 to 10 carbon atoms or is a saturated or unsaturated heterocyclic radical containing a hetero atom or a plurality of hetero atoms selected from among N, O, S in the molecule, which may be substituted by alkyl radicals or aryl radicals or be fused with cycloaliphatic or aromatic ring systems.

**4.** The process as claimed in claim 3, wherein Y is a radical derived from benzene, toluene, ethylbenzene, the isomeric xylene, from biphenyl, naphthalene, binaphthyl, benzyl or pyridine.

**5.** The process as claimed in any of claims 1 to 4, wherein the phosphorous triesters correspond to the formula

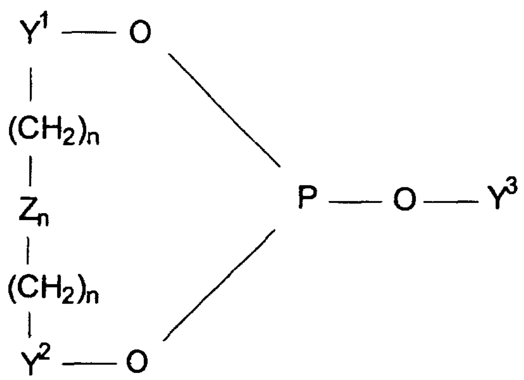
(2)

where $Y^1$, $Y^2$ and $Y^3$ are identical or different and are each an organic radical, in particular a radical derived from benzene, from naphthalene, from biphenyl or from binaphthyl, which may in each case be substituted by one or more alkyl radicals having from 1 to 20 carbon atoms, by one or more aryl, aralkyl, alkylaryl radicals having from 6 to 30 carbon atoms and/or by one or more cycloalkyl radicals having from 5 to 14 carbon atoms, by hydroxy groups and/or alkoxy radicals having from 1 to 10 carbon atoms and/or acid radicals ($-ac^-$), Z is a divalent bridging group, namely $-CR^1R^2-$, where $R^1$ and $R^2$ are, independently of one another, hydrogen or alkyl radicals having from 1 to 12 carbon atoms, -O-, -S-, -CO-, $-CH_2-CO-CH_2-$, in particular $-CH_2-$, -O-, -CO- and $-CH_2-CO-CH_2-$, and n are identical or different and are each 0 or 1 and when Z is $-CR^1R^2-$ are 1, 2 or 3, and the ester of the formula (2) contains at least one acid radical ($-ac^-$).

**6.** The process as claimed in any of claims 1 to 4, wherein the phosphorous triesters correspond to the formula

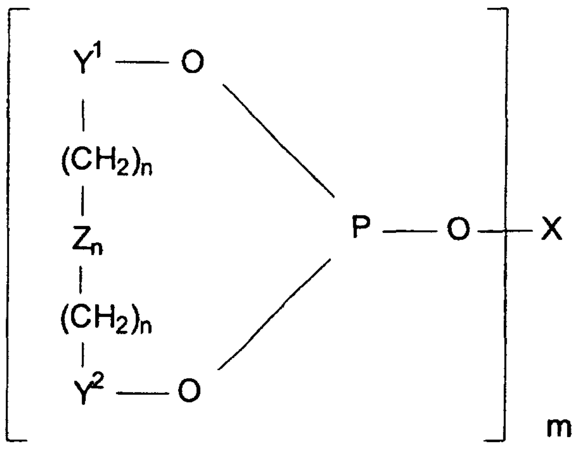

(3)

where $Y^1$ and $Y^2$ are identical or different and are each an organic radical, in particular a radical derived from benzene, from naphthalene, from biphenyl or from binaphthalene, which may in each case be substituted by one or more alkyl radicals having from 1 to 20 carbon atoms, by one or more aryl, aralkyl, alkylaryl radicals having from 6 to 30 carbon atoms and/or by one or more cycloalkyl radicals having from 5 to 14 carbon atoms, by hydroxy groups and/or alkoxy radicals having from 1 to 10 carbon atoms and/or acid radicals ($-ac^-$), Z is a divalent bridging group namely $-CR^1R^2-$, where $R^1$ and $R^2$ are, independently of one another, hydrogen or alkyl radicals having from 1 to 12 carbon atoms, -O-, -S-, -CO-, $-CH_2-CO-CH_2-$, in partiuclar $-CH_2-$, -O-, -CO- and - $CH_2-CO-CH_2-$, n are identical or different and are each 0 or 1, and when Z is $-CR^1R^2-$ are 1, 2 or 3, X is an m-valent bridging group selected from among alkylene radicals, alkyleneoxyalkylene radicals, arylene radicals and aryl-$Z_n$-aryl radicals, in particular an alkylene radical having from 2 to 18, preferably from 2 to 12, carbon atoms, and when X is aryl-$Z_n$-aryl Z is $-CH_2-CO-CH_2-$, where the radicals X may be substituted by one or more alkyl and/or alkoxy radicals and/or by one or more acid radicals ($-ac^-$) and the ester of the formula (3) contains at least one acid radical ($-ac^-$) and m is an integer from 2 to 6.

7. The process as claimed in any of claims 1 to 4, wherein the phosphorous triesters correspond to the fomula

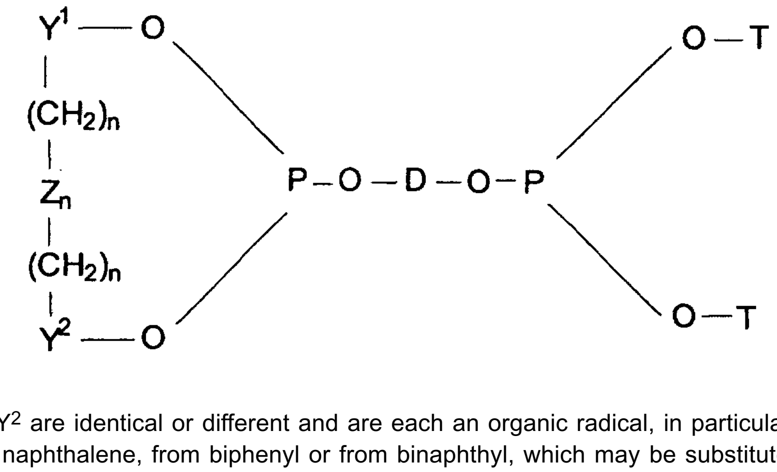

(4)

where $Y^1$ and $Y^2$ are identical or different and are each an organic radical, in particular a radical derived from benzene, from naphthalene, from biphenyl or from binaphthyl, which may be substituted by one or more alkyl radicals having from 1 to 20 carbon atoms, by one or more aryl, aralkyl, alkylaryl radicals having from 6 to 30 carbon atoms and/or by one or more cycloalkyl radicals having from 5 to 14 carbon atoms, by hydroxy groups and/or alkoxy radicals having from 1 to 10 carbon atoms and/or acid radicals ($-ac^-$), Z is a divalent bridging group, namely - $CR^1R^2-$, where $R^1$ and $R^2$ are, independently of one another, hydrogen or alkyl radicals having from 1 to 12 carbon atoms, -O-, -S-, -CO-, $-CH_2-CO-CH_2-$, in particular $-CH_2-$, -O-, -CO- and $-CH_2-CO-CH_2-$, n are identical or different and are each 0 or 1 and when Z is $-CR^1R^2-$ are 1, 2 or 3, D is a divalent hydrocarbon radical as bridging group, namely an alkylene radical having from 1 to 30 carbon atoms, an arylene, alkylarylene, arylalkylene radical having from 6 to 30 carbon atoms or an aryl-$Z_n$-aryl radical and T is a monovalent hydrocarbon radical having from

1 to 30 carbon atoms, and the ester of the formula (4) contains at least one acid radical (-ac⁻).

**8.** The process as claimed in any of claims 1 to 7, wherein at least from 3 to 300, preferably from 2 to 200 mol, of phosphorus(III) are used per mole of rhodium.

**9.** The process as claimed in any of claims 1 to 8, wherein the rhodium concentration, based on olefin used, is from 2 to 1000 ppm by weight, preferably from 3 to 400 ppm by weight and in particular from 5 to 100 ppm by weight.

**10.** The process as claimed in any of claims 1 to 9, wherein the reaction is carried out at temperatures of from 20 to 150°C, preferably from 80 to 140°C and in particular from 100 to 125°C.

**11.** The process as claimed in any of claims 1 to 10, wherein the reaction is carried out at pressures of from 0.1 to 20 MPa, preferably from 1 to 12 MPa and in particular from 3 to 7 MPa.

**12.** The process as claimed in any of claims 1 to 11, wherein olefins and olefin derivatives having from 2 to 20 carbon atoms are reacted.

## Revendications

**1.** Procédé en deux phases pour la préparation d'aldéhydes par transformation de monooléfines, de polyoléfines conjuguées et non conjuguées, de cyclooléfines ou de dérivés de ces classes de composés avec du monoxyde de carbone et de l'hydrogène à des températures de 20 à 150°C et des pressions de 0,1 à 20 MPa, **caractérisé par** la présence d'une phase organique contenant un aldéhyde et d'un liquide ionique non aqueux non soluble dans cette phase, de formule générale $(Q^+)_aA^{a-}$ avec au moins un composé du rhodium, **caractérisé en ce que** $Q^+$ représente des ions ammonium dérivés de monoamines de formule générale (5)

$$R^3R^4N^{\oplus} = CR^5R^6 \quad (5)$$

dans laquelle $R^3$, $R^4$, $R^5$ et $R^6$ sont identiques ou différents et représentent hydrogène, en particulier à condition qu'au moins un parmi $R^3$, $R^4$, $R^5$, $R^6$ ne représente pas hydrogène, ou un radical hydrocarboné aliphatique linéaire ou ramifié comprenant 1 à 20 atomes de carbone, un radical hydrocarboné cycloaliphatique ou aromatique comprenant 6 à 20 atomes de carbone ou un radical alcoxy comprenant 1 à 10 atomes de carbone ; ou représente des ions ammonium dérivés de monoamines de formule générale (6)

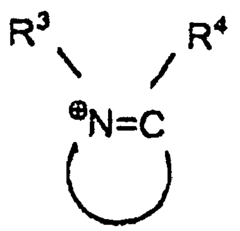

(6)

qui sont dérivés de composés cycliques saturés ou insaturés ainsi que de composée aromatiques avec à chaque fois un atome d'azote trivalent dans un cycle à 4 jusqu'à 10 membres, de préférence à 5 jusqu'à 6 membres, $R^3$, $R^4$ étant identiques ou différents et représentant hydrogène, un radical hydrocarboné aliphatique linéaire ou ramifié comprenant 1 à 20 atomes de carbone, un radical hydrocarboné cycloaliphatique ou aromatique comprenant 6 à 20 atomes de carbone ou un radical alcoxy comprenant 1 à 10 atomes de carbone ; ou représente des ions ammonium dérivés de diamines de formule générale (7) ou (8)

$$R^3R^4R^5N^{\oplus}\text{-}G\text{-}N^{\oplus}R^6R^7R^8 \quad (7)$$

$$R^3R^4N^{\oplus}=CR^5\text{-}G\text{-}R^5C=N^{\oplus}R^3R^4 \quad (8)$$

dans lesquelles $R^3$, $R^4$, $R^5$, $R^6$, $R^7$ et $R^8$ sont identiques ou différents et représentent hydrogène, un radical hydrocarboné aliphatique linéaire ou ramifié comprenant 1 à 20 atomes de carbone, un radical hydrocarboné cycloaliphatique ou aromatique comprenant 6 à 30 atomes de carbone, un radical alkylaryle comprenant 7 à 40 atomes de carbone ou un radical alcoxy comprenant 1 à 10 atomes de carbone, G représente un radical alkylène $(-CHR^9-)_d$, où $R^9$ représente hydrogène ou un radical hydrocarboné comprenant 1 à 5 atomes de carbone et d un nombre entier de 1 à 8, de préférence de 2 à 6, un radical arylène comprenant 6 à 30 atomes de carbone ou un radical alkylènearyle comprenant 7 à 40 atomes de carbone ; ou représente des cations dérivés d'imidazole et ses dérivés alkyle et phényle de formule générale (10)

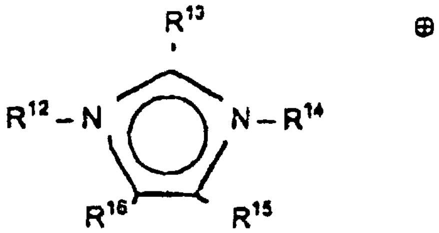

(10)

dans laquelle $R^{12}$, $R^{13}$, $R^{14}$, $R^{15}$ et $R^{16}$ sont identiques ou différents et représentent hydrogène, un radical alkyle en $C_1$ à $C_{30}$, un radical aryle en $C_6$ à $C_{40}$, un radical alkylaryle en $C_7$ à $C_{40}$, ou un radical $SiR_3^{17}$, dans lequel $R^{17}$ signifie un radical alkyle en $C_1$ à $C_{30}$ ou un radical aryle en $C_6$ à $C_{40}$, $A^{a-}$ représente l'anion d'un triester sulfoné ou carboxylé de l'acide phosphoreux et a est un nombre entier et égal à au moins 1 et la phase de produit contenant l'aldéhyde et la phase de catalyseur contenant le rhodium et le liquide ionique sont séparées l'une de l'autre par séparation des phases et la phase de catalyseur est recyclée partiellement ou totalement dans le processus d'hydroformylation.

2. Procédé selon la revendication 1, **caractérisé en ce que** le composant alcool du triester de l'acide phosphoreux correspond à la formule générale

$$(Qac)_b\text{-}Y\text{-}(OH)_c \qquad (1)$$

dans laquelle Y représente un radical organique, Q a la signification définie dans la revendication 1, ac représente un radical acide sulfonique ou acide carboxylique et b et c sont des nombres entiers et valent chacun au moins 1, c vaut en particulier 1 ou 2.

3. Procédé selon la revendication 2, **caractérisé en ce que** Y représente un radical aliphatique saturé linéaire ou ramifié, comprenant 1 à 20 atomes de carbone, qui peut être substitué par des radicaux hydroxy ou alcoxy comprenant 1 à 10 atomes de carbone, un radical cycloaliphatique saturé ou insaturé, monocyclique ou polycyclique comprenant 5 à 14 atomes de carbone dans le ou les cycles, un radical aromatique monocyclique ou polycyclique comprenant 6 à 14 atomes de carbone dans le ou les cycles, le radical cycloaliphatique et le radical aromatique pouvant à chaque fois être substitué par des radicaux alkyle comprenant 1 à 20 atomes de carbone, par des radicaux aryle, alkylaryle ou aralkyle comprenant 6 à 30 atomes de carbone, par des radicaux cycloalkyle comprenant 5 à 14 atomes de carbone, par des groupements hydroxy et par des radicaux alcoxy comprenant 1 à 10 atomes de carbone ou représente un radical hétérocyclique saturé ou insaturé présentant un hétéroatome ou plusieurs hétéroatomes du groupe N, O, S dans la molécule, qui est le cas échéant substitué par des radicaux alkyle ou aryle ou condensé avec des systèmes cycliques cycloaliphatiques ou aromatiques.

4. Procédé selon la revendication 3, **caractérisé en ce que** Y est un radical dérivé du benzène, du toluène, de l'éthylbenzène, des xylènes isomères, du biphényle, du naphtalène, du binaphtyle, du benzyle ou de la pyridine.

5. Procédé selon les revendications 1 à 4, **caractérisé en ce que** les triesters de l'acide phosphoreux correspondent à la formule générale

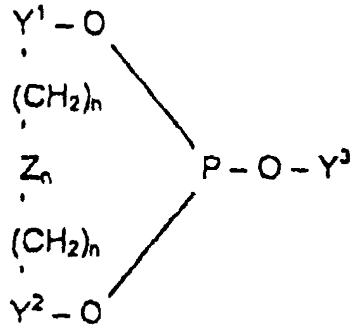

(2)

dans laquelle $Y^1$, $Y^2$, $Y^3$ sont identiques ou différents et représentent un radical organique, en particulier un radical dérivé du benzène, du naphtalène, du biphényle ou du binaphtyle, qui peut à chaque fois être substitué par un ou plusieurs radicaux alkyle comprenant 1 à 20 atomes de carbone, par un ou plusieurs radicaux aryle, aralkyle, alkylaryle comprenant 6 à 30 atomes de carbone et/ou par un ou plusieurs radicaux cycloalkyle comprenant 5 à 14 atomes de carbone, par des groupements hydroxy et/ou des radicaux alcoxy comprenant 1 à 10 atomes de carbone et/ou des radicaux acides ($-ac^-$), Z représente un groupement de pont divalent , à savoir $-CR^1R^2-$, $R^1$ et $R^2$ représentant indépendamment l'un de l'autre hydrogène ou des radicaux alkyle comprenant 1 à 12 atomes de carbone, -O-, -S-, -CO-, $-CH_2-CO-CH_2-$, en particulier $-CH_2-$, -O-, - CO- et $-CH_2-CO-CH_2-$ et n est identique ou différent et représente 0 ou 1 et, dans le cas où Z serait égal à - $CR^1R^2-$, n est égal à 1, 2 ou 3, et l'ester selon la formule (2) contient au moins un radical acide ($-ac^-$).

6. Procédé selon les revendications 1 à 4, **caractérisé en ce que** les triesters de l'acide phosphoreux correspondent à la formule générale

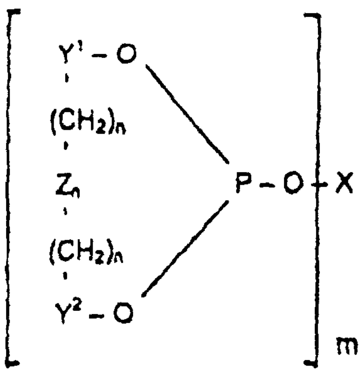

(3)

dans laquelle $Y^1$ et $Y^2$ sont identiques ou différents et représentent un radical organique, en particulier un radical dérivé du benzène, du naphtalène, du biphényle ou du binaphtyle, qui peut à chaque fois être substitué par un ou plusieurs radicaux alkyle comprenant 1 à 20 atomes de carbone, par un ou plusieurs radicaux aryle, aralkyle, alkylaryle comprenant 6 à 30 atomes de carbone et/ou par un ou plusieurs radicaux cycloalkyle comprenant 5 à 14 atomes de carbone, par des groupements hydroxy et/ou des radicaux alcoxy comprenant 1 à 10 atomes de carbone et/ou des radicaux acides ($-ac^-$), Z représente un groupement de pont divalent, à savoir $-CR^1R^2-$, $R^1$ et $R^2$ représentant indépendamment l'un de l'autre hydrogène ou des radicaux alkyle comprenant 1 à 12 atomes de carbone, -O-, -S-, -CO-, $-CH_2-CO-CH_2-$, en particulier $-CH_2-$, -O-, - CO- et $-CH_2-CO-CH_2-$, n est identique ou différent et représente 0 ou 1 et, dans le cas où Z serait égal à - $CR^1R^2-$, n est égal à 1, 2 ou 3 , X représente un groupement de pont de valence m du groupe des radicaux alkylène, alkylèneoxyalkylène, arylène ou aryl-$Z_n$-aryle, en particulier un radical alkylène comprenant 2 à 18, de préférence 2 à 12 atomes de carbone et dans la signification aryl-$Z_n$-aryle, Z représente $-CH_2-CO-CH_2-$, les radicaux désignés par X pouvant être substitués par un ou plusieurs radicaux alkyle et/ou alcoxy et/ou par un ou plusieurs radicaux acides ($-ac^-$) et l'ester selon la formule (3) contient au moins un radical acide ($-ac^-$) et m est un nombre entier de 2 à 6.

7. Procédé selon les revendications 1 à 4, **caractérisé en ce que** les triesters de l'acide phosphoreux correspondent à la formule générale

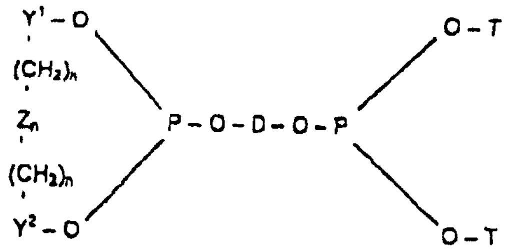

(4)

dans laquelle $Y^1$ et $Y^2$ sont identiques ou différents et représentent un radical organique, en particulier un radical dérivé du benzène, du naphtalène, du biphényle ou du binaphtyle, qui peut à chaque fois être substitué par un ou plusieurs radicaux alkyle comprenant 1 à 20 atomes de carbone, par un ou plusieurs radicaux aryle, aralkyle, alkylaryle comprenant 6 à 30 atomes de carbone et/ou par un ou plusieurs radicaux cycloalkyle comprenant 5 à 14 atomes de carbone, par des groupements hydroxy et/ou des radicaux alcoxy comprenant 1 à 10 atomes de carbone et/ou par des radicaux acides ($-ac^-$), Z représente un groupement de pont divalent, à savoir $-CR^1R^2-$, $R^1$ et $R^2$ représentant indépendamment l'un de l'autre hydrogène ou des radicaux alkyle comprenant 1 à 12 atomes de carbone, - O-, -S-, -CO-, $-CH_2-CO-CH_2-$, en particulier $-CH_2-$, -O-, - CO- et $-CH_2-CO-CH_2-$, n est identique ou différent et représente 0 ou 1 et, dans le cas de $-CR^1R^2-$, n est égal à 1, 2 ou 3, D est un radical hydrocarboné divalent servant de groupement de pont, à savoir un radical alkylène comprenant 1 à 30 atomes de carbone, un radical arylène, alkylarylène, arylalkylène comprenant 6 à 30 atomes de carbone et un radical aryl-$Z_n$-aryle et T représente un radical hydrocarboné monovalent comprenant 1 à 30 atomes de carbone et l'ester selon la formule (4) contient au moins un radical acide ($-ac^-$).

8. Procédé selon les revendications 1 à 7, **caractérisé en ce qu'**on utilise par mole de rhodium au moins 3 à 300, de préférence 2 à 200 moles de phosphore (III).

9. Procédé selon les revendications 1 à 8, **caractérisé en ce que** la concentration en rhodium par rapport à l'oléfine utilisée est de 2 à 1000 ppm en poids, de préférence de 3 à 400 ppm en poids et en particulier de 5 à 100 ppm en poids.

10. Procédé selon les revendications 1 à 9, **caractérisé en ce que** la transformation est réalisée à des températures de 20 à 150°C, de préférence de 80 à 140°C et en particulier de 100 à 125°C.

11. Procédé selon les revendications 1 à 10, **caractérisé en ce que** la transformation est réalisée à des pressions de 0,1 à 20 MPa, de préférence de 1 à 12 MPa et en particulier de 3 à 7 MPa.

12. Procédé selon les revendications 1 à 11, **caractérisé en ce qu'**on transforme des oléfines et des dérivés d'oléfines comprenant 2 à 20 atomes de carbone.